# EUROPEAN PATENT SPECIFICATION

(11) **EP 4 180 812 B1**
(45) Date of publication and mention of the grant of the patent: **25.02.2026**
(21) Application number: 21207832.3
(22) Date of filing: 11.11.2021
(51) Int. Cl.: G01N 33/574

(54) **METHOD FOR MONITORING THE RESPONSE TO NEOADJUVANT THERAPY FOR BREAST CANCER**
VERFAHREN ZUR ÜBERWACHUNG DES ANSPRECHENS AUF EINE NEOADJUVANSTHERAPIE FÜR BRUSTKREBS
PROCÉDÉ DE SURVEILLANCE DE LA RÉPONSE À UNE THÉRAPIE NÉOADJUVANTE POUR LE CANCER DU SEIN

(43) Date of publication of application: 17.05.2023
(73) Proprietor: Albert-Ludwigs-Universität Freiburg, 79098 Freiburg (DE)
(72) Inventor: SCHILLING, Oliver, 4052 Basel (CH); STEENBUCK, Derya Ines, 79085 Freiburg (DE); ERBES, Thalia, 79194 Gundelfingen (DE); WEIß, Daniela, 79312 Emmendingen (DE); JAEGER, Markus, 79238 Ehrenkirchen (DE); NOETHLING, Claudia, 79224 Umkirch (DE)
(74) Representative: Kraus & Lederer PartGmbB

(56) References cited:
- WO-A1-2018/081454
- M. SCHMIDT ET AL: "A Comprehensive Analysis of Human Gene Expression Profiles Identifies Stromal Immunoglobulin C as a Compatible Prognostic Marker in Human Solid Tumors", CLINICAL CANCER RESEARCH, vol. 18, no. 9, 20 February 2012 (2012-02-20), pages 2695 - 2703, XP055077875, ISSN: 1078-0432, DOI: 10.1158/1078-0432.CCR-11-2210
- KIM HYORI ET AL: "Prognostic implication of serum hepatocyte growth factor in stage II/III breast cancer patients who received neoadjuvant chemotherapy", JOURNAL OF CANCER RESEARCH AND CLINICAL ONCOLOGY, SPRINGER INTERNATIONAL, BERLIN, DE, vol. 142, no. 3, 18 November 2015 (2015-11-18), pages 707 - 714, XP035881611, ISSN: 0171-5216, [retrieved on 20151118], DOI: 10.1007/S00432-015-2072-5

## Description

### BACKGROUND OF THE INVENTION

Still, breast cancer represents the most common cancer type among women in Germany and worldwide. Every eighth woman in Germany gets the diagnosis of breast cancer in her lifetime (incidence of 165 women per 100 000). Even if the mortality rate has decreased over the last years, there is still a standardized death rate of 22,8, so that breast cancer is the fifth common cause of death in Germany. Moreover, between the age of 25 and 49 it is even the most common cause of death for women (5).

A curative therapy of breast cancer includes the removal of the cancer (R0 resection), the administration of cytostatic and if necessary, endocrine, and targeted antibody therapy. Moreover, a postoperative, local radiotherapy after breast-conserving surgery (BCS) is recommended (8).

Regarding overall survival neoadjuvant chemotherapy (NACT) is equivalent to adjuvant chemotherapy. Therapy-decision depends on subtype, grading, progress, and age of the patient. Indications for a NACT are lymphangiosis carcinomatosa, locally advanced carcinoma, inoperability. If the same chemotherapy regimen is recommended for adjuvant as neoadjuvant therapy, NACT should be given preference (8). However, NACT can lead to an improvement of operability, an increased rate of BCS, and in patients with triple-negative breast cancer (TNBC) and pathological complete remission (pCR), to an improvement of long-term clinical prognosis. To achieve pathological complete remission, response to NACT is essential (1).

Preoperative the effect of the cytotoxic therapy on the carcinoma is documented with the help of clinical examination and imaging such as sonography, MRI, PET-CT. An early response (a reduction in tumour size) to chemotherapy has proven to be a favourable marker for achieving a pCR (3). NACT offers the possibility to monitor response to possibly adjust the therapy regimen. Early discrimination between patients who respond to the therapy (responders) and those who do not respond (non-responders) is therefore essential for being able to adapt therapy individually (4).

Since the clinical methods to monitor response to neoadjuvant breast cancer therapy, preferably NACT, described are either dependent on the examiner and based on a tumour size reduction, which sometimes occurs with some delay, or not able to reliably distinguish responders from non-respondents with a sufficient degree of certainty and/or at an early stage in the NACT, there is a need for a further method which can help to distinguish responders from non-respondents at an early stage.

Schmidt et al. (2012) Clinical Cancer Research 18(9), 2695-2703 identifies immunoglobulin kappa C (IGKC) as a diagnostic marker for risk stratification in human cancer.

WO 2018/081454 A1 relates to biomarkers for identifying chemo-resistant tumor cells, such as triple-negative breast cancers (TNBCs), and methods of treating a subject having said chemo-resistant TNBCs.

Kim et al. (2016) Journal of cancer research and clinical oncology, 142, 707-714 describes the prognostic implication of serum hepatocyte growth factor in stage II/III breast cancer patients after receiving neoadjuvant chemotherapy.

### SUMMARY OF THE INVENTION

The present inventors investigated serum samples of breast cancer patients and found that the proteins N-cadherin, hepatocyte growth factor receptor (HGFR), centrosomal protein of 192 kDa (Cep192), contactin-1, and cholinesterase displayed changes in serum protein level in breast cancer patients undergoing neoadjuvant breast cancer therapy, relative to the level in a comparative sample. Surprisingly, these changes were indicative of a subject being a responder or a non-responder to neoadjuvant breast cancer therapy. Remarkably, such changes in serum protein level for some marker proteins could be observed as early as 4 to 6 weeks after start of the neoadjuvant breast cancer therapy, thus allowing for discrimination between responders and non-responders to neoadjuvant breast cancer therapy.

Similarly, the present inventors found that serum protein levels of certain immunoglobulin-components changed in serum from patients undergoing neoadjuvant breast cancer therapy, relative to a comparative sample, which changes surprisingly were also indicative of the subject being a responder or a non-responder to neoadjuvant breast cancer therapy.

Thus, determination of the level of the five marker proteins N-cadherin, HGFR, Cep192, contactin-1, and cholinesterase, as well as the immunoglobulin-components, in the serum samples has the advantage of allowing for a specific discrimination between responders and non-responders to neoadjuvant breast cancer therapy, early in the course of the neoadjuvant treatment regimen and with good consistency.

Furthermore, the present inventors surprisingly found that the finding of a subject being a responder using one or more of the above-mentioned marker proteins was predictive of the subject, after completion of neoadjuvant breast cancer therapy, preferably NACT, achieving a pathological complete remission (pCR).

The present invention is defined in the appended claims.

### BRIEF DESCRIPTION OF THE DRAWINGS

**Figure 1****:** Methodology applied for serum proteome analysis.
**Figure 2****:** Comparison of T1 to T0 in complete responders (CR). The proteins show significant changes in their relative intensity (red dots).
**Figure 3****:** Comparison of T1 to T0 in Non-Responders (NR). The proteins show significant changes in their relative intensity (red dots).
**Figure 4****:** Comparison of T3 and T0 in CR. The proteins show significant changes in their relative intensity (red dots).
**Figure 5****:** Comparison of T3 to T0 in NR. The proteins show significant changes in their relative intensity (red dots).
**Figure 6****:** Relative intensity (arbitrary units) of the HGF receptor (HGFR) over at time in comparison to the control group.
**Figure 7****:** Relative Intensity (arbitrary units) of N-cadherin over time compared to the control group.
**Figure 8****:** Comparison NR at T1 vs CR at T1 (arbitrary units); significant change (log(FC)=log fold change) in relative intensity (red dot)
**Figure 9****:** Relative Intensity (arbitrary units) of centrosomal protein 192 kDa (Cep192) over time in comparison to the control group.
**Figure 10****:** Relative Intensity (arbitrary units) of contactin-1 over time compared to the control group.
**Figure 11****:** Relative Intensity (arbitrary units) of cholinesterase over time compared to the control group.

### DETAILED DESCRIPTION

The present invention relates to methods for determining whether a subject is a responder or a non-responder to breast cancer therapy, in particular to neoadjuvant breast cancer therapy; and for predicting a favorable outcome of a cancer therapy, in particular neoadjuvant breast cancer therapy, especially for predicting whether or not a subject, after completion of neoadjuvant breast cancer therapy, is likely to achieve a pathological complete remission (pCR).

In a first aspect, the present invention relates to a method for determining whether a subject is a responder or a non-responder to neoadjuvant breast cancer therapy comprising determining the level of one or more marker proteins selected from the group consisting of N-cadherin; hepatocyte growth factor receptor (HGFR); centrosomal protein of 192 kDa (Cep192); contactin-1; cholinesterase; one or more immunoglobulin-components, preferably selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61; and combinations thereof, wherein the one or more marker proteins comprises HGFR,
a) in one or more serum samples of a subject undergoing neoadjuvant breast cancer therapy; and
b) in a comparative serum sample of that subject, or a comparative pooled serum sample from healthy subjects, or a comparative pooled serum sample from subjects suffering from breast cancer;
wherein a change in the level of the one or more marker proteins in one or more serum samples of a), relative to the level of the respective one or more marker proteins in the comparative serum sample of b), is indicative of the subject being a responder or a non-responder to neoadjuvant breast cancer therapy.

As understood by those skilled in the art, by increasing the number of the above marker proteins, for which the level in the serum samples of a) and b) is to be determined using the methods of the invention, the certainty of the subject indeed being responsive or not responsive (i.e. of "of the subject being a responder or a non-responder") to the neoadjuvant breast cancer therapy, preferably the NACT, may increase. According to a particular embodiment, the term "indicative" means that there is an at least 75 %, preferably at least 80 %, more preferably at least 85 %, even more preferably at least 90 %, even more preferably at least 95 %, or 96 %, or 97%, or 98%, or 99 %, or 99.5 %, or 99.9 %, or 99.99 %, or 99.999 % certainty (likelihood) of the subject being responsive to the neoadjuvant breast cancer therapy, preferably the NACT.

The methods of the invention described herein are carried out in vitro. The term "in vitro" has its usual meaning in the art, preferably referring to methods that are carried out on serum samples outside the body of the subject from whom the serum samples were obtained.

Herein, a "subject" or "patient" is preferably a human subject or patient, more preferably a female human subject or patient. The subject is a "cancer patient," i.e. one who is suffering from one or more symptoms of breast cancer. According to a preferred embodiment the subject is human, preferably a female human.

The terms "cancer" and "cancerous" are known in the art and typically refer to or describe the physiological condition in mammals in which a population of cells are characterized by unregulated cell growth. An "advanced" cancer is one which has spread outside the site or organ of origin, either by local invasion or metastasis. Accordingly, the term "advanced" cancer includes both locally advanced and metastatic disease. The term "invasive" cancer refers to cancer that has spread beyond the primary tumour in which it developed, and is growing in surrounding, healthy tissues. Invasive cancer is sometimes referred to as infiltrating cancer. A "locally recurrent" cancer is cancer that returns after treatment in the same place as a previously treated cancer. "Early-stage breast cancer" as used herein preferably refers to breast cancer that has not spread beyond the breast or the axillary lymph nodes. Such cancer is generally treated with neoadjuvant or adjuvant therapy. "Metastatic" cancer refers to cancer which has spread from one part of the body (e.g. the breast) to another part of the body.

According to one embodiment, the subject suffers from locally advanced breast cancer (LABC). "Locally advanced breast cancer/carcinoma" or "LABC", which preferably in the absence of distant metastasis, comprises tumours more than 5 cm in size with regional lymphadenopathy (N1-3), and/or tumours of any size with direct extension to the chest wall or skin, or both, regardless of regional lymphadenopathy; and/or is characterized by the presence of regional lymphadenopathy (clinically fixed or matted axillary lymph nodes, or any of infraclavicular, supraclavicular, or internal mammary lymphadenopathy) regardless of tumour stage.

The term "breast cancer" is known in the art and preferably refers to a cancer that starts in the breast, usually in the inner lining of the milk ducts or lobules. There are different types of breast cancer, with different stages (spread), aggressiveness, and genetic makeup. The classification of breast cancer (see e.g. (8)) includes a pathological assessment of the size, infiltration into the surrounding tissue (lymphatic system, vessels, perineural sheaths) and distant metastasis. Furthermore, the histological degree of differentiation and the proliferation fraction (Ki67 expression) are important factors in assessing the prognosis and the response to systemic therapy. Using immunohistochemical staining (estrogen, progesterone, androgen receptor positivity), breast cancer can be divided into hormone receptor negative and positive subtypes; this can be used to adapt the therapy. The Her2 status even enables targeted antibody therapy in the case of Her2 positivity.

For example, Union International Cancer Control (UICC) TNM Classification of Malignant Tumours, 8th Edition, December 2016, Wiley (ISBN: 978-1-119-26357-9*)*, or the same from the American Joint Committee on Cancer (AJCC), is suitable as a standard for classifying the extent of spread of breast cancer in a subject and for determining the breast cancer stage (cancer staging).

Histological grading involves classification into three grades (Grades I, II, and III), wherein the tumour cells for Grade I (GI) are well differentiated; Grade II (GII) are moderately differentiated; Grade III (GIII) are poorly differentiated (8).

Classification of breast cancer into molecular subtypes using biomarkers is known in the art, and includes for example determination whether the breast cancer is progesterone receptor positive (PR+), or progesterone receptor negative (PR-), e.g. as detected by immunohistochemistry or ligand binding assays; whether the breast cancer is estrogen receptor positive (ER+), or estrogen receptor negative (ER-), e.g. as detected by immunohistochemistry or ligand binding assays; whether the breast cancer is Human epidermal growth factor receptor 2 (HER2) positive (HER2+), or HER2 negative (HER2-), e.g. as detected by observable HER2 gene amplification after in situ hybridization; and whether the protein Ki-67 is expressed at high levels (Ki-67high), or at low levels. The term "negative as used above, may refer to the absence or a reduction of the respective biomarker abundance/expression, as e.g. compared to healthy tissue. A classification standard has been published for example in Goldhirsch et al, Annals of Oncology 24: 2206-2223, 2013 *(see also (8)).*

Molecular subtypes of breast cancer are known in the art and include for example Luminal A breast cancer (e.g., ER+ and/or PR+, HER2-, Ki-67low, optionally GI) breast cancer; Luminal B, [HER2 positive] breast cancer (e.g., ER+, and/or PR+, HER2+, Ki67high or Ki67low, optionally GII); Luminal B [HER2 negative] breast cancer (e.g., ER+ and/or PR+, HER2-, and and Ki67high, optionally GII) breast cancer; triple negative (basal-like) breast cancer (e.g., ER-, PR-, HER-; optionally GIII); or HER2 positive (non-luminal) breast cancer (HER2-enriched breast cancer) (e.g., ER-, PR-, HER+, optionally GIII). Additionally, normal-like breast cancer *(e.g., ER+ and*/*or PR+, HER2-, Ki-67low, GI-III)* may be a further subtype.

Breast cancer subtypes may be categorized on an immunohistochemical basis. The breast cancer may be invasive ductal carcinoma, ductal carcinoma in situ, or invasive lobular carcinoma. Preferably for histological classification the 2019 WHO Classification of Tumours of the Breast is used.

Histological subtypes of breast cancer include, but are not limited to, invasive ductal carcinoma (IDC) "not otherwise specified" and IDC subtypes (e.g. mixed, pleomorphic, osteoclast types); invasive lobular carcinoma (ILC); Paget's disease of the nipple; inflammatory breast cancer; phyllodes tumours of the breast; tubular carcinoma; medullary carcinoma; alveolar carcinoma; solid variant carcinoma; signet ring cell carcinoma; metaplastic carcinoma; mucinous carcinoma; neuroendocrine tumours; oncocytic subtypes; invasive papillary carcinoma; cribriform carcinoma; or invasive apocrine carcinoma, locally advanced breast cancer; or metastatic breast cancer.

In certain implementations, the subject suffers from breast cancer selected from the group consisting of lymphangiosis carcinomatosa, locally advanced carcinoma/cancer (LABC), and inoperable breast cancer.

In another embodiment, the subject suffers from Luminal A breast cancer; Luminal B [human epidermal growth factor receptor 2-positive] (LumB[HER2+]) breast cancer; Luminal B [human epidermal growth factor receptor 2-negative] (LumB[HER2-]) breast cancer; triple negative breast cancer (TNBC); human epidermal growth factor receptor 2 (HER2) positive (non-luminal) breast cancer; or normal-like breast cancer. In another embodiment, the subject suffers from triple negative breast cancer (TNBC); Luminal B [human epidermal growth factor receptor 2-negative] (LumB[HER2-]) breast cancer; or human epidermal growth factor receptor 2 (HER2) positive (non-luminal) breast cancer; preferably TNBC or LumB[HER2-] breast cancer.

In yet another embodiment, the subject suffers from invasive ductal carcinoma (IDC) "not otherwise specified" and IDC subtypes (e.g. mixed, pleomorphic, osteoclast types); invasive lobular carcinoma (ILC); Paget's disease of the nipple; inflammatory breast cancer; phyllodes tumours of the breast; tubular carcinoma; medullary carcinoma; alveolar carcinoma; solid variant carcinoma; signet ring cell carcinoma; metaplastic carcinoma; mucinous carcinoma; neuroendocrine tumours; oncocytic subtypes; invasive papillary carcinoma; cribriform carcinoma; or invasive apocrine carcinoma; locally advanced breast cancer; or metastatic breast cancer; or combinations thereof.

The terms "responder" and non-responder" as used herein in the context of cancer therapy, particularly breast cancer therapy, more particularly neoadjuvant breast cancer therapy, signify that the subject is responsive or not responsive, respectively, to the cancer therapy, particularly the breast cancer therapy, more particularly the neoadjuvant breast cancer therapy.

Responsiveness of a "responder" to (neoadjuvant) breast cancer therapy, in terms of an observable decrease in the size of one or more tumours and/or lesions, and/or in the extent of the cancer in the breast, and/or a decrease in the cancer stage, may be apparent already at the time of sample collection of one or more of the one or more serum samples, or may only become apparent by (certain) other examination methods at a later time point during the course of treatment. Responsiveness to therapy may be documented during neoadjuvant breast cancer therapy, preferably NACT, for example using breast ultrasound. In rarer cases a responsiveness may be documented by means of computed tomography (CT), magnetic resonance imaging (MRI), or mammography. After completion of (neoadjuvant) breast cancer therapy, preferably NACT, responsiveness to therapy may be documented for example by the assessment of the resected material as carried out by a pathologist.

According to one embodiment, a "responder" to (neoadjuvant) breast cancer therapy is characterized by a decrease in the size of one or more tumours or lesions (e.g. a greater than 30 % reduction in the (preferably) greatest tumour diameter), or in the extent of the cancer in the breast (e.g. based on extent of spread and cancer staging, based on location/spread in the breast and lymph nodes or based on histological grading or histologically classification; for example using one or more parameter of the UICC/AJCC TNM staging system), or a decrease in the cancer stage (e.g. as determined by the UICC/AJCC TNM staging system), in response to treatment. In another embodiment, a "responder" is characterized by a decrease in tumour size or cancer stage. In yet another embodiment, a "responder" is characterized by a greater than 30 % reduction in (preferably) the greatest tumour diameter.

According to one embodiment, a responder is either a "complete responder" or a "partial responder". The term "complete responder" refers to a subject showing a complete response to the breast cancer therapy, particularly the neoadjuvant breast cancer therapy, i.e. preferably referring to an absence of invasive and in situ residuals in breast and regional lymph nodes (e.g. no clinical evidence of tumour in the breast and regional lymph nodes, and/or ypTO (and preferably also ypNO) in the UICC (or AJCC) TNM staging system). More preferably a complete responder refers to a subject having achieved "pathologic complete remission (pCR)" as further specified below, for example, as assessed on resected material (preferably resected after completion of (neoadjuvant) breast cancer therapy, preferably neoadjuvant chemotherapy (NACT)).

The term "partial responder" refers to a subject showing a partial response to the breast cancer therapy, particularly the neoadjuvant breast cancer therapy, i.e. preferably referring to a decrease in the size of one or more tumours or lesions (e.g. a greater than 30 % reduction in the (preferably) greatest tumour diameter), or in the extent of the cancer in the breast (e.g. based on extent of spread and cancer staging, based on location/spread in the breast and lymph nodes or based on histological grading or histologically classification; for example using one or more parameter of the UICC/AJCC TNM staging system), or a decrease in the cancer stage (e.g. as determined by the UICC/AJCC TNM staging system), in response to treatment, but no complete response as defined above. Alternatively, a partial responder is characterized by a greater than 30 % reduction in the (preferably) greatest tumour diameter, but no complete response as defined above.

The term "pathologic complete remission", abbreviated as "pCR", and also referred to as "pathologic complete response" as used in the context of the present invention is not particularly limited. In the context of the present invention, the term "pCR" in breast cancer may, for example, be defined as the absence of invasive and in situ residuals in breast and regional lymph nodes (or "absence of residual invasive and in situ breast cancer"), i.e., the complete eradication of all invasive and noninvasive cancer, for example, as assessed on resected material (preferably resected after completion of (neoadjuvant) breast cancer therapy, preferably neoadjuvant chemotherapy (NACT)).

According to a preferred definition, the term "pCR" in the context of the present invention is defined as the absence of residual invasive and in situ breast cancer, preferably on hematoxylin and eosin evaluation of a complete resected breast specimen and all sampled regional lymph nodes, preferably following completion of neoadjuvant breast cancer therapy (i.e., ypTO ypNO in the UICC (or AJCC) TNM staging system). The term "pCR" in breast cancer, in the context of the present invention, may thus preferably be understood as referring to a lack of all signs of cancer in tissue samples removed during surgery or biopsy (no tumor/ malignant cells can be detected in the resected material), as a consequence of the neoadjuvant breast cancer therapy.

According to a specific embodiment of the present invention, the finding of the subject being a responder using the method of the present invention is predictive of the subject, after completion of neoadjuvant breast cancer therapy, achieving a pathological complete remission (pCR). As understood by those skilled in the art, by increasing the number of the above marker proteins, for which the level in the serum samples of a) and b) is to be determined using the methods of the present invention, the predictive value (or the likelihood) that a responder will achieve a pCR after completion of neoadjuvant breast cancer therapy (preferably NACT), may increase. According to specific embodiment, the term "predictive" means that if the subject is found to be a responder, there is an at least 70 %, preferably at least 75 %, more preferably at least 80 %, even more preferably at least 85 %, even more preferably at least 90 %, even more preferably at least 95 %, or 96 %, or 97%, or 98%, or 99 % likelihood of the subject, after completion of neoadjuvant breast cancer therapy, achieving a pathological complete remission (pCR).

According to a particular embodiment, a "non-responder" to (neoadjuvant) breast cancer therapy is characterized by absence of a decrease in the size (e.g. a less than 30 % reduction) of any of the tumours or lesions, or of the greatest tumour and/or lesion (e.g. a less than 30 % reduction); in the extent of the cancer in the breast e.g. based on extent of spread and cancer staging, based on location/spread in the breast and lymph nodes or based on histological grading or histologically classification; for example using one or more parameter of the UICC/AJCC TNM staging system); and in the cancer stage (e.g. as determined by the UICC/AJCC TNM staging system). In another embodiment, a "non-responder" is characterized by absence of a decrease in tumour size and stage. In yet another embodiment, a "non-responder" is characterized by a stable disease (i.e. preferably a reduction of less than 30 % or an increase of up to 20 % in the (preferably) greatest tumour diameter) or by a progressive disease (i.e. preferably an increase of more than 20 % in the (preferably) greatest tumour diameter or the appearance of new disease).

The term "breast cancer therapy" is not particularly limited and may for example refer to administration of an anti-tumour agent and/or radiation therapy. As used herein an "anti-tumour agent" refers to an active ingredient or drug used to treat cancer. Non-limiting examples of anti-tumour agents herein include chemotherapy agents, HER dimerization inhibitors, HER antibodies, antibodies directed against tumour associated antigens, anti-hormonal compounds, cytokines, EGFR- targeted drugs, anti-angiogenic agents, tyrosine kinase inhibitors, growth inhibitory agents and antibodies, cytotoxic agents, antibodies that induce apoptosis, COX inhibitors, farnesyl transferase inhibitors, antibodies that binds oncofetal protein CA 125, HER2 vaccines, Raf or ras inhibitors, liposomal doxorubicin, topotecan, taxane, dual tyrosine kinase inhibitors, TLK286, EMD-7200, pertuzumab, trastuzumab, erlotinib, and bevacizumab.

A "chemotherapy" preferably refers to the use of a chemical compound useful in the treatment of cancer. Examples of chemotherapeutic agents, used in chemotherapy, include alkylating agents such as thiotepa and CYTOXAN^{®} cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; TLK 286 (TELCYTA^{™}); acetogenins (especially bullatacin and bullatacinone); delta-9-tetrahydrocannabinol (dronabinol, MAPJNOL^{®}); beta-lapachone; lapachol; colchicines; betulinic acid; a camptothecin (including the synthetic analogue topotecan (HYCAMTIN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; bisphosphonates, such as clodronate; antibiotics such as the enediyne antibiotics (e. g. , calicheamicin, especially calicheamicin gamma II and calicheamicin omegall (see, e.g., Agnew, Chem Intl. Ed. Engl., 33: 183-186 (1994)) and anthracyclines such as annamycin, AD 32, alcarubicin, daunorubicin, doxorubicin, dexrazoxane, DX-52-1, epirubicin, GPX-100, idarubicin, valrubicin, KRN5500, menogaril, dynemicin, including dynemicin A, an esperamicin, neocarzinostatin chromophore and related chromoprotein enediyne antiobiotic chromophores, aclacinomysins, actinomycin, authramycin, azaserine, bleomycins, cactinomycin, carabicin, carminomycin, carzinophilin, chromomycinis, dactinomycin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin, liposomal doxorubicin, and deoxydoxorubicin), esorubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, and zorubicin; folic acid analogues such as denopterin, pteropterin, and trimetrexate; purine analogs such as fludarabine, 6- mercaptopurine, thiamiprine, and thioguanine; pyrimidine analogs such as ancitabine, azacitidine, 6- azauridine, carmofur, cytarabine, dideoxyuridine, doxifluridine, enocitabine, and floxuridine; androgens such as calusterone, dromostanolone propionate, epitiostanol, mepitiostane, and testolactone; anti- adrenals such as aminoglutethimide, mitotane, and trilostane; folic acid replenisher such as folinic acid (leucovorin); aceglatone; anti-folate anti-neoplastic agents such as ALIMTA^{®}, LY231514 pemetrexed, dihydrofolate reductase inhibitors such as methotrexate, anti-metabolites such as 5-fluorouracil (5-FU) and its prodrugs such as UFT, S-1 and capecitabine, and thymidylate synthase inhibitors and glycinamide ribonucleotide formyltransferase inhibitors such as raltitrexed (TOMUDEX , TDX); inhibitors of dihydropyrimidine dehydrogenase such as eniluracil; aldophosphamide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK7 polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; arabinoside ("Ara-C"); cyclophosphamide; thiotepa; taxanes; chloranbucil; gemcitabine (GEMZAR^{®}); 6-thioguanine; mercaptopurine; platinum; platinum analogs or platinum-based analogs such as cisplatin, oxaliplatin and carboplatin; vinblastine (VELBAN^{®}); etoposide (VP- 16); ifosfamide; mitoxantrone; vincristine (ONCOVIN^{®}); vinca alkaloid; vinorelbine (NAVELBINE^{®}); novantrone; edatrexate; daunomycin; aminopterin; xeloda; ibandronate; topoisomerase inhibitor RFS 2000; difluorometlhylomithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN^{™}) combined with 5-FU and leucovorin.

An anti-hormonal agent preferably refers to an active ingredient or drug that acts to regulate or inhibit hormone action on tumours such as anti-estrogens and selective estrogen receptor modulators (SERMs), including, for example, tamoxifen (including NOLVADEX^{®} tamoxifen), raloxifene, droloxifene, 4- hydroxytamoxifen, trioxifene, keoxifene, LY1 17018, onapristone, and FARESTON^{®} toremifene; aromatase inhibitors; and anti-androgens such as flutamide, nilutamide, bicalutamide, leuprolide, and goserelin; as well as troxacitabine (a 1,3-dioxolane nucleoside cytosine analog); antisense oligonucleotides, particularly those that inhibit expression of genes in signaling pathways implicated in abherant cell proliferation, such as, for example, PKC -alpha, Raf, H-Ras, and epidermal growth factor receptor (EGF-R); vaccines such as gene therapy vaccines, for example, ALLOVECTIN^{®} vaccine, LEUVECTIN^{®} vaccine, and VAXID^{®} vaccine; PROLEUKIN^{®} rIL-2; LURTOTECAN^{®} topoisomerase 1 inhibitor; ABARELIX^{®} rmRH; and pharmaceutically acceptable salts, acids or derivatives of any of the above.

The term "cytotoxic agent" as used herein preferably refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

The term "neoadjuvant breast cancer therapy" (or "neoadjuvant breast cancer treatment") is not particularly limited and refers to a treatment, preferably the administration of a therapeutic agent (i.e. an anti-tumour agent), before/prior to the primary treatment for the disease (preferably surgery), in order to enhance the outcome of primary treatment and to reduce the risk of breast cancer recurrence. In the context of the present invention, the term "primary treatment" refers to a treatment, which is aimed at completely removing the cancer from the body or kill all the cancer cells. The primary treatment in the context of the present invention is preferably breast surgery, e.g. lumpectomy or mastectomy.

The neoadjuvant breast cancer therapy is not particularly limited and may for example comprises, or consists of, neoadjuvant chemotherapy (NACT); neoadjuvant endocrine therapy; neoadjuvant immunotherapy; and/or neoadjuvant radiotherapy. The neoadjuvant breast cancer therapy may comprises, or consists of, administration of anthracyclines such as doxorubicin (adriamycin), epirubicin, daunorubicin, valrubicin, idarubicin, mitoxantrone, daunorubicin and dexrazoxan; taxanes such as paclitaxel, docetaxel, nab paclitaxel, and cabazitaxel; cyclophosphamid; fluoropyrimidines, such as 5-fluorouracil and 5-deoxyfluorouridine; methotrexate; Lapatinib; Trastuzumab; Bevacizumab; Pertuzumab; ixabepilone; eribulin; platinum analog or platinum-based analog such as carboplatin; radiotherapy; and endocrine therapy, such as tamoxifen, goserelin acetate, aromatase inhibitors such as anastrozole, exemestane and letrozole, fulvestrant; and combinations thereof; which is optionally followed by surgery.

According to a preferred embodiment the "neoadjuvant breast cancer therapy" comprises, or consists of, neoadjuvant chemotherapy (NACT), which is optionally followed by surgery of the tumour. The duration of the NACT is not particularly limited and depends in particular on the choice of therapeutic(s), and in particular the choice of the chemotherapeutic or combination of chemotherapeutics. The NACT may last for e.g. 16 to 28 weeks, preferably 18 to 24, more preferably for about 20 to 24 weeks. The term "about" as used herein means that the specified numerical value or range also includes an increase or decrease of 1 from the stated numerical value or range, or alternative an increase or decrease of 10 % from the stated numerical value or range. NACT is, in particular, indicated, if the subject suffers from breast cancer selected from the group consisting of lymphangiosis carcinomatosa, locally advanced carcinoma/cancer (LABC), and inoperable breast cancer.

The neoadjuvant chemotherapy (NACT) is not particularly limited and may comprises, or consists of, administration of an anthracycline, such as doxorubicin (adriamycin), epirubicin, daunorubicin, valrubicin, idarubicin, mitoxantrone, daunorubicin and dexrazoxan preferably epirubicin, optionally together with cyclophosphamide and/or a fluoropyrimidine, in combination or sequentially administered with a taxane, such as paclitaxel, docetaxel, nab paclitaxel, and cabazitaxel, preferably paclitaxel (or, instead of a taxane alone, a combination of a taxane, like paclitaxel, with a platinum analog or platinum-based analog such as cisplatin, oxaliplatin and carboplatin, preferably carboplatin, for example in case of TNBC). This NACT may be combined with an immunotherapeutic, such as Trastuzumab, or Pertuzumab, or a combination of Trastuzumab and Pertuzumab (e.g., for Her2+ tumors), administered concomitantly for part of the duration of the NACT.

The anthracycline(s), optionally together with cyclophosphamide and/or a fluoropyrimidine, may be administered at the start of the NACT, for example one dose every 1, 2, 3, 5, 6, 7, or 8,... weeks, preferably either every 2 or every 3 weeks, preferably for 2 to 8 doses, more preferably for 3 to 6 doses, even more preferably for 4 doses. This is preferably followed by the administration of a taxane (or as specified above, instead of a taxane alone, a combination of taxane, like paclitaxel, with a platinum analog/platinum-based analog such as carboplatin, for example in case of TNBC), such as paclitaxel, preferably one dose per week, for preferably 5 to 20, more preferably 10 to 15, even more preferably 11, 12, or 13, even more preferably for 12, doses; or alternatively one dose every 2, 3, 4, 5, or 6 weeks, preferably every 2, 3, or 4 weeks, for preferably 2 to 10, more preferably 3 to 6, even more preferably for 3, 4, or 5 doses.

The term "one dose every (or per) 1/2/3/4/etc. weeks" means that a dose comprising one or more than one drugs (active ingredients) is administered every 1/2/3/4/etc. weeks. Preferably, the complete dose is administered at the start of the 1/2/3/4/etc. weeks, followed by no administration/treatment for the rest of the 1/2/3/4/etc. weeks. This is preferably repeated on a regular schedule. Herein, the terms "after a (first/second/third/etc.) dose" and "after administration of a (first/second/third/etc.) dose" are used interchangeably and preferably refer to a timepoint after said (first/second/third/etc.) dose has been completely administered to a subject. After completion of administration of said first/second/third/etc. dose and before administration of the subsequent dose, there preferably is a period of no treatment/administration (i.e. a period of rest) of e.g. 1/2/3/4/etc. weeks. Herein, the terms "before a (first/second/third/etc.) dose" and "before administration of a (first/second/third/etc.) dose" are used interchangeably and preferably refer to a timepoint prior to administration of said (first/second/third/etc.) dose to a subject.

The term "cycle" or "treatment cycle" is known to those skilled in the art and as used herein in the context of cancer treatment or therapy preferably refers to a period of time of specific duration, usually comprising or consisting of a period of treatment (that might for example last from a few hours to a few days) followed by a period of rest (no treatment). One cycle might have a duration of e.g. 1 to 6 weeks, preferably about 1, 2, 3 or 4 weeks, depending on the drug(s) and the specific treatment plan. Such a cycle is repeated on a regular schedule. Repeating the cycle multiple times on a regular schedule, makes up a course of treatment (also called "cycle of treatment").

The neoadjuvant breast cancer therapy, preferably the NACT, may comprise, or consists of, administration of a first drug (active ingredient) or set of drugs, for example for 2 to 10, preferably 2 to 8, more preferably for 3 to 6, e.g. for 3, or for 4, doses (the duration between administration of one dose and the next dose being for example 1, 2, 3 or 4 weeks, preferably 2 or 3 weeks, i.e. one dose every 1, 2, 3 or 4 weeks, preferably every 2 or 3 weeks); followed by administration of a second drug or set of drugs, for example for 2 to 16, preferably for 4 to 15, more preferably for 8 to 15, even more preferably for 10 to 15, e.g. for 12 doses (the duration between administration of one dose and the next dose being for example 1, 2, 3 or 4 weeks, preferably 1 or 2 weeks, i.e one dose every 1, 2, 3 or 4 weeks, preferably every 1 or 2 weeks).

According to a specific embodiment of the present invention, the neoadjuvant breast cancer therapy, preferably the NACT, comprises, or consists of, administration of epirubicin (preferably 90 mg/m²) and/or cyclophosphamide (preferably 600 mg/m²), and paclitaxel (preferably 80 mg/m²), administered in combination or sequentially; preferably involving administration of epirubicin and/or cyclophosphamid one dose either every two or every three weeks, preferably for 3 to 6, more preferably for 4, doses, followed by administration of paclitaxel preferably one dose per week, preferably for 10 to 15, more preferably for 12, doses.

According to a further specific embodiment the NACT comprises, or consists of, administration of epirubicin (preferably 90 mg/m²) and cyclophosphamid (preferably 600 mg/m²) one dose either every two or every three weeks for 4 doses, followed by administration of paclitaxel (preferably 80 mg/m²) one dose per week for 12 doses.

The term "serum sample" refers to a sample which comprises or consists of serum. For determining the level of one or more marker proteins in a serum sample, the serum sample is typically processed by procedures known in the art.

The method for determining the level of one or more marker proteins in the serum samples is not particularly limited, and may for example include liquid chromatography-tandem mass spectrometry (LC-MS/MS), e.g. high pH reversed-phase high performance liquid chromatography (RP-HPLC) followed by tandem mass spectrometry; and/or an immunoassay such as enzyme-linked immunosorbent assay (ELISA); and/or western blot; and/or targeted quantitative proteomics.

Optionally, before determining the protein level, for example by LC-MS/MS, the serum samples are depleted of one or more, preferably at least five, more preferabl at least seven, even more preferably 10 to 60, e.g. 14, high-abundance serum proteins. For this, for example a commercially available immunoaffinity kit can be used.

According to a preferred embodiment of the present invention liquid chromatography-tandem mass spectrometry (LC-MS/MS), preferably high pH reversed-phase liquid chromatography followed by tandem mass spectrometry for detection, is used for determining the level of the one or more marker proteins. Before liquid chromatography-tandem mass spectrometry (LC-MS/MS), but after the optional depletion of high-abundance serum proteins, the proteins in the serum may be digested by one or more proteases, e.g. Lys-C and/or Trypsin, and desalted. This may be followed by isobaric labelling.

According to the first aspect of the present invention, the one or more marker proteins is selected from the group consisting of N-cadherin; hepatocyte growth factor receptor (HGFR); centrosomal protein of 192 kDa (Cep192); contactin-1; cholinesterase; one or more immunoglobulin-components, preferably selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61; and combinations thereof, and wherein the one or more marker protein comprises HGFR.

The term "N-cadherin" refers to a protein also known as cadherin-2 (CDH2), neural cadherin (NCAD), CD325, or CDw325, which in human is encoded by the gene CDH2, and may be further identified by the UniProt-ID P19022. The term "Hepatocyte growth factor receptor (HGFR)" refers to a protein also known as c-Met, tyrosine-protein kinase Met, or proto-oncogene c-Met, which in humans is encoded by the MET gene, and may be further identified by the UniProt-ID P08581. The term "centrosomal protein of 192 kDa (Cep192)" refers to a protein, which in humans is encoded by the CEP192 gene, and may be further identified by the UniProt-ID Q8TEP8. Contactin-1, also known as CNTN1, is a protein which in humans is encoded by the CNTN1 gene, and may be further identified by the UniProt-ID Q12860. The term "cholinesterase" refers to a protein also known as acylcholine acylhydrolas, butyrylcholine esterase, choline esterase II, or pseudocholinesterase, which in humans is encoded by the BCHE gene, and may be further identified by the UniProt-ID P06276.

The term immunoglobulin-component as used herein preferably refers to a protein, which is a component of an immunoglobulin, such as IgG, IgM, IgA and IgE. The term "Immunoglobulin kappa constant" refers to a protein, which in human is encoded by the gene IGKC, and may be further identified by the UniProt-ID P01834. The term "Immunoglobulin kappa variable 2-30" refers to a protein, which in human is encoded by the gene IGKV2-30, and may be further identified by the UniProt-ID P06310. The term "Immunoglobulin lambda-like polypeptide 5" refers to a protein, which in human is encoded by the gene IGLL5, and may be further identified by the UniProt-ID B9A064. The term "Ig-like domain-containing protein" refers to protein, which may be further identified by the UniProt-ID A0A0J9YY99. The term "Immunoglobulin heavy constant mu" refers to a protein, which in human is encoded by the gene IGHM, and may be further identified by the UniProt-ID P01871. The term "Immunoglobulin kappa variable 2-40" refers to a protein, which in human is encoded by the gene IGKV2-40, and may be further identified by the UniProt-ID A0A087WW87. The term "Immunoglobulin lambda variable 7-46" refers to a protein, which in human is encoded by the gene IGLV7-46, and may be further identified by the UniProt-ID A0A075B6I9. The term "Immunoglobulin lambda variable 8-61" refers to a protein, which in human is encoded by the gene IGLV8-61, and may be further identified by the UniProt-ID A0A075B6I0.

The one or more marker proteins comprises HGFR; and optionally N-Cadherin, Cep192, contactin-1, and/or cholinesterase; and/or one or more immunoglobulin-components, preferably selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61. According to yet another embodiment of the present invention, the one or more marker proteins comprises HGFR; and optionally N-Cadherin, Cep192, contactin-1 and/or cholinesterase.

According to yet another embodiment of the present invention, the one or more marker proteins comprises N-cadherin and HGFR; and optionally Cep192, contactin-1, and/or cholinesterase; and/or one or more immunoglobulin-components, preferably selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61. According to yet another embodiment of the present invention, the one or more marker proteins comprises N-cadherin and HGFR; and optionally Cep192, contactin-1 and/or cholinesterase.

According to yet another embodiment of the present invention, the one or more marker proteins further comprises one or more immunoglobulin-components, preferably selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61; and optionally N-Cadherin; and/or optionally Cep192, contactin-1 and/or cholinesterase. According to yet another embodiment of the present invention, the one or more marker proteins further comprises one or more immunoglobulin-components selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and andlmmunoglobulin lambda variable 8-61.

According to yet another embodiment of the present invention, the one or more marker proteins further comprises Cep192, contactin-1 and/or cholinesterase, for example Cep192 and contactin-1; or contactin-1 and cholinesterase; or Cep192 and cholinesterase; or Cep192, contactin-1 and cholinesterase, and optionally N-cadherin.

According to further specific embodiments of the present invention the one or more marker proteins consists of HGFR

According to further specific embodiment of the present invention the one or more marker proteins may consist of N-cadherin and HGFR; or of HGFR and Cep192; or of HGFR and contactin-1; or of HGFR and cholinesterase; or of N-cadherin, HGFR and Cep192; or of N-cadherin, HGFR and contactin-1; or of N-cadherin, HGFR and cholinesterase; or of HGFR, Cep192 and contactin-1; or of HGFR, Cep192 and cholinesterase; or of HGFR, contactin-1 and cholinesterase; or of N-cadherin, HGFR, Cep192 and contactin-1; or of N-cadherin, HGFR, Cep192 and cholinesterase; or of N-cadherin, HGFR, contactin-1 and cholinesterase; or of HGFR, Cep192, contactin-1 and cholinesterase; or of one or more immunoglobulin-components, preferably selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61 in addition to the above marker proteins in the above combinations.

The above-mentioned embodiments with specific combinations of the different marker proteins are examples of combinations of these marker proteins exemplified for illustrative purposes and should not be construed as limiting the possible combinations of the above-mentioned marker proteins which are in accordance with the present invention. The present invention of course also covers embodiments with combinations of these marker proteins not exemplified above.

The level of one or more marker proteins is determined a) in one or more serum samples of a subject undergoing neoadjuvant breast cancer therapy; and b) in a comparative serum sample of that subject, or in a comparative pooled serum sample from healthy subjects, or in a comparative pooled serum sample from subjects suffering from breast cancer. The serum samples of a) and b), before their use in the methods of the present invention, may for example be sampled and stored in a manner which ensures/maximizes the stability of the serum proteins comprised in the serum samples.

The one or more serum sample of a) (i.e. the one or more serum samples of a subject undergoing neoadjuvant breast cancer therapy), may comprise one, or two, or three, or four, or even more serum samples.

If the marker protein comprises at least one of N-cadherin, HGFR, Immunoglobulin kappa constant and Immunoglobulin lambda-like polypeptide 5, and at least one of Cep192, contactin-1, cholinesterase, Ig-like domain-containing protein, Immunoglobulin heavy constant mu, Immunoglobulin kappa variable 2-40, Immunoglobulin lambda variable 7-46 and Immunoglobulin lambda variable 8-61, the one or more serum samples of a) preferably comprises at least two serum samples (e.g. exactly two): at least one for determining the level of N-cadherin, HGFR, Immunoglobulin kappa constant and/or Immunoglobulin lambda-like polypeptide 5 preferably taken at an earlier timepoint relative to the start of the neoadjuvant breast cancer therapy (as early as e.g. about 2 to 6 week after, or about 4 to 6 weeks after, start of the neoadjuvant breast cancer therapy); and at least one for determining the level of Cep192, contactin-1, cholinesterase, Ig-like domain-containing protein, Immunoglobulin heavy constant mu, Immunoglobulin kappa variable 2-40, Immunoglobulin lambda variable 7-46 and/or Immunoglobulin lambda variable 8-61, taken at a later timepoint relative to the start of the neoadjuvant breast cancer therapy (e.g. about 12 to 18 weeks after start of the neoadjuvant breast cancer therapy).

Thus, according to a preferred embodiment of the first aspect of the method of the present invention, if the marker protein is N-Cadherin or HGFR, a serum sample of a) is a sample taken about 2 to 12 weeks, preferably about 2 to 10 weeks, more preferably about 2 to 8 weeks, even more preferably about 2 to 7 weeks, even more preferably about 2 to 6 weeks, even more preferably about 4 to 6 weeks, after start of the neoadjuvant breast cancer therapy. According to another preferred embodiment of the first aspect of the method of the present invention, if the marker protein is Cep192, contactin-1, or cholinesterase, the serum samples of a) is a sample taken about 6 to 22 weeks (or preferably about 6 to 20 weeks, or preferably about 6 to 18 weeks), preferably about 8 to 22 weeks, more preferably about 8 to 20 weeks, more preferably about 8 to 18 weeks, even more preferably about 9 to 18 weeks, even more preferably 10 to 18 weeks, even more preferably 11 to 18 weeks, even more preferably 12 to 18 weeks, even more preferably 13 to 18 weeks, even more preferably about 14 to 18 weeks, after start of the neoadjuvant breast cancer therapy.

Furthermore, if the marker protein is Immunoglobulin kappa constant or Immunoglobulin lambda-like polypeptide 5, preferably a serum sample of a) is a sample taken about 2 to 12 weeks, preferably about 2 to 10 weeks, more preferably about 2 to 8 weeks, even more preferably about 2 to 7 weeks, even more preferably about 2 to 6 weeks, even more preferably about 4 to 6 weeks, after start of the neoadjuvant breast cancer therapy. That is, for Immunoglobulin kappa constant or Immunoglobulin lambda-like polypeptide 5 as a marker protein a sample may be preferably used which was taken as early as e.g. 2 to 6 week after start of the neoadjuvant breast cancer therapy.

If the marker protein is Ig-like domain-containing protein, Immunoglobulin heavy constant mu, Immunoglobulin kappa variable 2-40, Immunoglobulin lambda variable 7-46, or Immunoglobulin lambda variable 8-61, preferably the serum samples of a) is a sample taken about 6 to 22 weeks (or preferably about 6 to 20 weeks, or preferably about 6 to 18 weeks), preferably about 8 to 22 weeks, more preferably about 8 to 20 weeks, even more preferably about 8 to 18 weeks, even more preferably about 9 to 18 weeks, even more preferably 10 to 18 weeks, even more preferably 11 to 18 weeks, even more preferably about 12 to 18 weeks, even more preferably about 13 to 18 weeks, even more preferably about 14 to 18 weeks, after start of the neoadjuvant breast cancer therapy. That is, for Ig-like domain-containing protein, Immunoglobulin heavy constant mu, Immunoglobulin kappa variable 2-40, Immunoglobulin lambda variable 7-46, or Immunoglobulin lambda variable 8-61 as a marker protein, a sample may be preferably used which was taken later, e.g. 12 to 18 week after start of the neoadjuvant breast cancer therapy.

If the marker protein is Immunoglobulin kappa variable 2-30, preferably the serum samples of a) is a sample taken about 2 to 22 weeks, preferably about 2 to 20 weeks, more preferably about 2 to 18 weeks, more preferably about 2 to 16 weeks, even more preferably about 2 to 18 weeks, for example about 2 to 6 weeks or about 4 to 6 weeks, or about 12 to 18 weeks or about 14 to 18 weeks, after start of the neoadjuvant breast cancer therapy. That is, for Immunoglobulin kappa variable 2-30 as a marker protein a sample may be preferably used which was taken as early as e.g. 4 to 6 week after start of the neoadjuvant breast cancer therapy, or which was taken later for example about 12 to 18 weeks after start of the neoadjuvant breast cancer therapy.

A serum sample of a) is a serum sample taken after start (i.e. after administration of a first dose) of neoadjuvant breast cancer therapy, preferably NACT. According to a preferred embodiment of the method of first aspect of the present invention, if the marker protein is N-Cadherin, HGFR, Immunoglobulin kappa constant, or Immunoglobulin lambda-like polypeptide 5, a serum sample of a) is a sample taken after a first dose (i.e. after administration of a first dose of the neoadjuvant breast cancer therapy) but before a fifth dose (i.e. before administration of a fifth dose); preferably after a second dose (i.e. after administration of a second dose) but before a fifth dose; more preferably after administration of a second dose but before administration of a fourth dose, or after administration of a first dose but before administration of a third dose; more preferably after administration of a second dose but before administration of a fourth dose; even more preferably after administration of a second dose but before administration of a third dose, e.g. just prior to administration of a the third dose, of neoadjuvant breast cancer therapy, preferably NACT.

According to another preferred embodiment of the first aspect of the method of the present invention, if the marker protein is Cep192, contactin-1, cholinesterase, Ig-like domain-containing protein, Immunoglobulin heavy constant mu, Immunoglobulin kappa variable 2-40, Immunoglobulin lambda variable 7-46, or Immunoglobulin lambda variable 8-61, a serum sample of a) is a sample taken after a fourth dose (i.e. after administration of the fourth dose) but before the twentieth dose (i.e. before administration of the twentieth dose); or after a fourth dose (i.e. after administration of the fourth dose) but before the eighteenth dose (i.e. before administration of the eighteenth dose); preferably after administration of the fifth dose but before administration of the eighteenth dose; more preferably after administration of the fifth dose but before administration of the seventeenth dose, or after administration of the fifth dose but before administration of the sixteenth dose; even more preferably after administration of the fifth dose but before administration of the fifteenth dose, or after administration of the sixth dose but before administration of the fifteenth dose; even more preferably after administration of the fifth dose but before the administration of the fourteenth dose, or after administration of the sixth dose but before the administration of the fourteenth dose; even more preferably after administration of the sixth dose but before the administration of the thirteenth dose; even more preferably after administration of the seventh dose but before the administration of the thirteenth dose; even more preferably after administration of the seventh dose but before administration of the twelfth dose; even more preferably after administration of the seventh dose but before administration of the eleventh dose; even more preferably after administration of the eighth dose but before administration of the twelfth dose; even more preferably after administration of the eighth dose but before administration of the eleventh dose; even more preferably after administration of the nineth dose but before administration of the eleventh dose; even more preferably after administration of the tenth dose but before the eleventh dose (i.e. before administration of the eleventh dose), e.g. just prior administration of the eleventh dose, of neoadjuvant breast cancer therapy, preferably NACT.

According to another preferred embodiment of the first aspect of the method of the present invention, if the marker protein is Immunoglobulin kappa variable 2-30, a serum sample of a) is a sample taken after the first dose (i.e. after administration of the first dose) but before the twentieth dose (i.e. before administration of the twentieth dose); preferably after administration of the first dose but before administration of the eighteenth dose; more preferably after administration of the second dose but before administration of the seventeenth dose, or after administration of the second dose but before the sixteenth dose; more preferably after administration of the second dose but before administration of the fifteenth dose; even more preferably after administration of the second dose but before administration of the fourteenth dose; even more preferably after administration of the second dose but before administration of the thirteenth dose; even more preferably after administration of the second dose but before administration of the twelfth dose; even more preferably after administration of the second dose but before administration of the eleventh dose, e.g. just prior to administration of the third dose or just prior to administration of the eleventh dose, of neoadjuvant breast cancer therapy, preferably NACT.

Additionally, if the neoadjuvant breast cancer therapy comprises or consists of NACT, for example, of an anthracycline (e.g. administered every two or three weeks), preferably epirubicin, optionally in combination with cyclophosphamide and/or a fluoropyrimidine, in combination or preferably sequentially administered with a taxane (e.g. administered weekly), preferably paclitaxel, and if the marker protein is N-Cadherin or HGFR, preferably a serum sample of a) is a sample taken taken just before administration of a third dose of NACT. Moreover, if the neoadjuvant breast cancer therapy comprises or consists of NACT, for example, of an anthracycline (e.g. administered every two or three weeks), preferably epirubicin, optionally in combination with cyclophosphamide and/or a fluoropyrimidine, in combination or preferably sequentially administered with a taxane (e.g. administered weekly), preferably paclitaxel, and if the marker protein is Cep192, contactin-1, or cholinesterase, preferably a serum sample of a) is a sample taken taken just before administration of a seventh dose of NACT.

Additionally, if the neoadjuvant breast cancer therapy comprises or consists of NACT, for example, of an anthracycline (e.g. administered every two or three weeks), preferably epirubicin, optionally in combination with cyclophosphamide and/or a fluoropyrimidine, in combination or preferably sequentially administered with a taxane (e.g. administered weekly), preferably paclitaxel, and if the marker protein is Immunoglobulin kappa constant or Immunoglobulin lambda-like polypeptide 5, preferably a serum sample of a) is a sample taken taken just before administration of a third dose of NACT.

Additionally, if the neoadjuvant breast cancer therapy comprises or consists of NACT, for example, of an anthracycline (e.g. administered every two or three weeks), preferably epirubicin, optionally in combination with cyclophosphamide and/or a fluoropyrimidine, in combination or preferably sequentially administered with a taxane (e.g. administered weekly), preferably paclitaxel, and if the marker protein is Ig-like domain-containing protein, Immunoglobulin heavy constant mu, Immunoglobulin kappa variable 2-40, Immunoglobulin lambda variable 7-46, or Immunoglobulin lambda variable 8-61, preferably a serum sample of a) is a sample taken taken just before administration of an eleventh dose of NACT.

Additionally, if the neoadjuvant breast cancer therapy comprises or consists of NACT, for example, of an anthracycline (e.g. administered every two or three weeks), preferably epirubicin, optionally in combination with cyclophosphamide and/or a fluoropyrimidine, in combination or preferably sequentially administered with a taxane (e.g. administered weekly), preferably paclitaxel, and if the marker protein is Immunoglobulin kappa variable 2-30, preferably a serum sample of a) is a sample taken at a timepoint between just before administration of a third to just before administration of an eleventh dose of NACT, e.g. just before administration of a third dose of NACT or just before administration of an eleventh dose of NACT.

The term "a serum samples of a)" as used herein refers to a serum sample of the "one or more serum samples of a subject undergoing neoadjuvant breast cancer therapy" under point a) of the methods of the present invention.

The comparative serum sample of b) is preferably a comparative serum sample of that subject, or a comparative pooled serum sample from healthy subjects, The term "of that subjects" as used in this context refers to the subject tested with the inventive method for determining whether a subject is a responder or a non-responder to neoadjuvant breast cancer therapy. The term "pooled sample" refers to a sample, which is obtain by mixing at least two samples, e.g. from different subjects, together. In the context of the present invention, the term "pooled serum sample" preferably refers to a serum sample, which is obtain by mixing together serum samples from at least two subjects, preferably at least five subjects, more preferably at least ten subjects, e.g. about 20 to 30 subjects. The term "healthy subjects" as used in the present context is known by the skilled person, and preferably refers to subjects not suffering from breast cancer. In one embodiment, the comparative serum sample of b) is a comparative pooled sample from healthy subjects.

In a preferred embodiment, the comparative serum sample of b) is a comparative serum sample of that subject. If the comparative serum sample of b) is a comparative serum sample of that subject, the comparative serum sample of b) is preferably a sample taken before or at the start of the neoadjuvant breast cancer therapy. This means that the sample may have been taken, for example, some days prior (e.g., 1-10, or 1-7, or 1-5, or 1-3 days prior), or just prior to, or just after the start, of the neoadjuvant breast cancer therapy, i.e. for example 1-7 days prior, or on the same day when the neoadjuvant breast cancer therapy is started, just before or just after the first therapeutic dose is administered. According to a preferred embodiment, the comparative serum sample of b) is a sample taken before, preferably just prior to, e.g. on the same day as, the start of the neoadjuvant breast cancer therapy (i.e. just before administration of the first dose of neoadjuvant breast cancer therapy, preferably NACT).

According to a particular embodiment wherein the one or more marker proteins comprises N-cadherin, the comparative serum sample of b) is a comparative pooled serum sample from healthy subjects. According to an alternative embodiment the comparative serum sample of b) is a comparative pooled serum sample from subjects suffering from breast cancer. Preferably the term "subjects suffering from breast cancer" in this context refers to subjects suffering from a similar, more preferably the same, type of breast cancer as the subject tested with the inventive method for determining whether a subject is a responder or a non-responder to neoadjuvant breast cancer therapy.

According to the method of the first aspect of the present invention, a change in the level of the one or more marker proteins in one or more serum samples of a), relative to the level of the respective one or more marker proteins in the comparative serum sample of b), is indicative of the subject being a responder or a non-responder to neoadjuvant breast cancer therapy. A change preferably means an increase or a decrease in the level of the one or marker proteins of a sample of a) relative to the comparative sample of b).

In the methods of the present invention, an increase may mean for example that the level of the one or more marker protein in the serum sample(s) of a) is decreased by at least 25 %, or by at least 30 %, or by at least 35 %, or by at least 40 %, or by at least 50 %, or by at least 60 %, or by at least 75 %, or by at least 85 %, relative to the control level, i.e. the level of the respective marker proteins in the comparative sample. A decrease may mean for example that the level of the one or more marker protein in the serum sample(s) of a) is increased by at least 25 %, or by at least 30 %, or by at least 35 %, or by at least 50 %, or by at least 75 %, or by at least 100 %, or by at least 150 %, or by at least 200 %, relative to the control level, i.e. the level of the respective marker proteins in the comparative sample. In the methods of the present invention, the level of the one or more marker protein in the serum sample(s) of a) may be constant, i.e., preferably changing (increase or decrease) by less than 50 %, or by less than 35 %, or by less than 30 %, or by less than 25 %, or by less than 20 %, relative to the control level, i.e. the level of the respective marker proteins in the comparative sample.

According to a preferred embodiment of the present invention, relative to the level of the respective protein marker in the comparative serum sample of b), an increase in the protein level of N-cadherin in a serum samples of a) is indicative of the subject being a responder, and a decrease or a constant protein level is indicative of the subject being a non-responder. According to a further preferred embodiment, relative to the level of the respective protein marker in the comparative serum sample of b), a decrease in the protein level of HGFR in a serum samples of a) is indicative of the subject being a responder; and a constant protein level or an increase is indicative of the subject being a non-responder.

According to a further preferred embodiment of the present invention, a decrease in the protein level of Cep192 in a serum samples of a) is indicative of the subject being a responder, and a constant protein level or an increase is indicative of the subject being a non-responder. According to a further preferred embodiment, a constant protein level of contactin-1 in a serum samples of a) or an increase is indicative of the subject being a responder, and a decrease is indicative of the subject being a non-responder. According to yet another preferred embodiment a constant protein level of cholinesterase in a serum samples of a) or a decrease is indicative of the subject being a responder, and an increase is indicative of the subject being a non-responder.

According to a further preferred embodiment of the invention, relative to the level of the respective protein marker in the comparative serum sample of b), a decrease in the protein level of one or more immunoglobulin components, preferably selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, Immunoglobulin lambda variable 8-61, and combinations thereof, in one or more serum samples of a) is indicative of the subject being a responder, and a constant protein level or an increase is indicative of the subject being a non-responder.

According to a further preferred embodiment, the subject is
- a "responder" if relative to the level of the respective protein marker in the comparative serum sample of b), the protein level of N-cadherin in a serum sample of a) is increased by at least 25 %, preferably by at least 30 %, more preferably by at least 35 %, or by at least 40 %, or by at least 45 %, or by at least 50 %, or by at least 60 % or by at least 75 %, or by at least 100 %, or by at least 150 %, or by at least 200 %; and/or
- is a non-responder if relative to the level of the respective protein marker in the comparative serum sample of b), the protein level of N-cadherin in a serum sample of a) is decreased by at least 25 % (preferably by at least 30 %, more preferably by at least 35 %, or by at least 40 %, or by at least 45 %, or by at least 50 %, or by at least 60 % or by at least 70 %, or by at least 80 %, or by at least 90 %), or is constant (which preferably means a change (increase or decrease) of less than 50 %, more preferably of less than 40 %, even more preferably of less than 35 %, or of less than 30 %, or of less than 25 %, or of less than 20 %), relative to the level of the respective protein marker in the comparative serum sample of b).

According to a further preferred embodiment, the subject is
- a "responder", if relative to the level of the respective protein marker in the comparative serum sample of b), the protein level of HGFR and/or Cep192 in a serum sample of a) is decreased by at least 25 %, preferably by at least 30 %, more preferably by at least 35 %, or by at least 40 %, or by at least 45 %, or by at least 50 %, or by at least 60 % or by at least 70 %, or by at least 80 %, or by at least 90 %; and/or
- a "non-responder", if relative to the level of the respective protein marker in the comparative serum sample of b), the protein level of HGFR and/or Cep192 in a serum sample of a) is constant, which preferably means a change (increase or decrease) of less than 50 %, more preferably of less than 40 %, even more preferably of less than 35 %, or of less than 30 %, or of less than 25 %, or of less than 20, relative to the level of the respective protein marker in the comparative serum sample of b), or is increased (e.g. by at least 25 %, or by at least 30 %, or by at least 35 %, or by at least 40 %, or by at least 45 %, or by at least 50 %, or by at least 60 % or by at least 75 %, or by at least 100 %, or by at least 150 %, or by at least 200 %).

According to a further preferred embodiment, the subject is
- a "responder", if relative to the level of the respective protein marker in the comparative serum sample of b), the protein level of cholinesterase in a serum sample of a) is constant, which preferably means a change (increase or decrease) of less than 50 %, more preferably of less than 40 %, even more preferably of less than 35 %, or of less than 30 %, or of less than 25 %, or of less than 20, relative to the level of the respective protein marker in the comparative serum sample of b), or is decreased (e.g. by at least 25 %, or by at least 30 %, more or by at least 35 %, or by at least 40 %, or by at least 45 %, or by at least 50 %, or by at least 60 % or by at least 70 %, or by at least 80 %, or by at least 90 %); and/or
- a "non-responder", if relative to the level of the respective protein marker in the comparative serum sample of b), the protein level of cholinesterase in a serum sample of a) is increased by at least 25 %, preferably by at least 30 %, more preferably by at least 35 %, or by at least 40 %, or by at least 45 %, or by at least 50 %, or by at least 60 % or by at least 75 %, or by at least 100 %, or by at least 150 %, or by at least 200 %.

According to a further preferred embodiment, the subject is
- a "responder", if relative to the level of the respective protein marker in the comparative serum sample of b), the protein level of contactin-1 in a serum sample of a) is constant, which preferably means a change (increase or decrease) of less than 50 %, more preferably of less than 40 %, even more preferably of less than 35 %, or of less than 30 %, or of less than 25 %, or of less than 20 %, relative to the level of the respective protein marker in the comparative serum sample of b), or is increased (e.g. by at least 25 %, or by at least 30 %, or by at least 35 %, or by at least 40 %, or by at least 45 %, or by at least 50 %, or by at least 60 % or by at least 75 %, or by at least 100 %, or by at least 150 %, or by at least 200 %); and/or
- a "non-responder", if relative to the level of the respective protein marker in the comparative serum sample of b), the protein level of contactin-1 in a serum sample of a) is decreased by at least 25 %, preferably by at least 30 %, more preferably by at least 35 %, or by at least 40 %, or by at least 45 %, or by at least 50 %, or by at least 60 % or by at least 70 %, or by at least 80 %, or by at least 90 %.

According to a yet a further preferred specific embodiment, the subject is
- a "responder", if relative to the level of the respective protein marker in the comparative serum sample of b), the protein level of the one or more immunoglobulin components in one or more serum samples of a) decreases by at least 25 %, preferably by at least 30 %, more preferably by at least 35 %, or by at least 40 %, or by at least 45 %, or by at least 50 %, or by at least 60 % or by at least 70 %, or by at least 80 %, or by at least 90 %; and/or
- a "non-responder", if relative to the level of the respective protein marker in the comparative serum sample of b), the protein level of the one or more immunoglobulin components in the one or more serum samples of a) is constant, which preferably means a change (increase or decrease) of less than 50 %, more preferably of less than 40 %, even more preferably of less than 35 %, or of less than 30 %, or of less than 25 %, or of less than 20, relative to the level of the respective protein marker in the comparative serum sample of b), or is increased (e.g. by at least 25 %, or by at least 30 %, or by at least 35 %, or by at least 40 %, or by at least 45 %, or by at least 50 %, or by at least 60 % or by at least 75 %, or by at least 100 %, or by at least 150 %, or by at least 200 %).

### EXAMPLES

### Introduction

Liquid biopsies are a very promising diagnostic method because of their low clinical expenditure of timepoint and their low invasiveness. Here on the molecular pathological level a marker is sought as an indicator of therapy response.

In the following study, serum was preserved as a liquid biopsy and proteins were examined for potential markers.

### Methods

In a retrospective longitudinal cohort study, serum samples from patients suffering from breast cancer of the subtype Luminal B (Her2neu / -) and TNBC were examined. The 22 patients received 4x epirubicin (90 mg / m2) / cyclophosphamide (600 mg / m2) every three or two weeks, followed by 12x paclitaxel (80 mg / m2) in weekly intervals (see Tables 1 and 2). Following neoadjuvant chemotherapy (NACT), the carcinomas were excised and examined histopathological so that the sera obtained were classified into complete responders (CR) and non-responders post-op (NR).

**Table 1: Patients of group CR**

| **Complete responders (CR)** | | | | | | |
|---|---|---|---|---|---|---|
| **Sample** | **Cancer subtype** | **CTx-scheme** | **ypT** | **ypN** | **G** | **age** |
| L1_t1 | LumB[HER2-] | 4x EC/ 12x P | ypTO | ypNO (0/10) | G3 | 40 |
| L3_t1 | LumB[HER2-] | EC dense | ypTO | ypNO (0/2) | G3 | 63 |
| L5_t1 | LumB[HER2-] | EC dense | ypTO | ypNO | G3 | 65 |
| L7_t1 | LumB[HER2-] | 4x EC/ 12x P | ypTO | ypNO (0/1) | G2 | 54 |
| L9_t1 | LumB[HER2-] | EC dense | ypTO | ypNO | G2 | 66 |
| L11_t1 | LumB[HER2-] | 4x EC/ 12x P | ypTO | ypNO (0/1) | G3 | 61 |
| L13_t1 | LumB[HER2-] | 4x EC/ 12x P | ypTO | ypNOsn (0/1) | G3 | 36 |
| L15_t1 | LumB[HER2-] | 4x EC/ 12x P | ypTO | ypNO (0/2) | G3 | 49 |
| T1_t1 | TNBC | EC dense | ypTO | ypNO | G3 | 40 |
| T3_t1 | TNBC | EC dense | ypTO | ypNO | G3 | 66 |
| T5_t1 | TNBC | 4x EC/ 12x P | ypTO | ypNO(sn) (0/2) | G3 | 48 |

That is, in the context of this study a subject/patient was classified as a CR (or complete responder) in case of achieving pathological complete remission (i.e. = [ypTO and ypN0]) upon completion of the neoadjuvant breast cancer therapy, in particular NACT, as assessed post-operatively after completion of therapy.

**Table 2: Patients of group NR**

| **Non responder (NR)** | | | | | | |
|---|---|---|---|---|---|---|
| **Sample** | **Cancer Subtype** | **CTx-scheme** | **ypT** | **ypN** | **G** | **age** |
| L2_t1 | LumB[HER2-] | 4x EC/ 12x P | ypT1c | ypNO (0/4) | G2 | 33 |
| L4_t1 | LumB[HER2-] | EC dense | ypT1c | ypN1a(sn) (2/2) | G2 | 45 |
| L6_t1 | LumB[HER2-] | EC dense | ypT2 | ypN3a(16/17) | G2 | 30 |
| L8_t1 | LumB[HER2-] | 4x EC/ 12x P | ypT1b | ypN1a(sn) (2/2) | G3 | 49 |
| L10_t1 | LumB[HER2-] | EC dense | ypT3 | ypN2 | G2 | 46 |
| L12_t1 | LumB[HER2-] | 4x EC/ 12x P | ypT4b | ypN1a (2/24) | G2 | 43 |
| L14_t1 | LumB[HER2-] | 4x EC/ 12x P | ypT2a | ypNO (0/3) | G3 | 66 |
| L16_t1 | LumB[HER2-] | 4x EC/ 12x P | ypT1c | ypN2a (5/12) | G3 | 51 |
| T2_t1 | TNBC | EC dense | ypT2 | ypN1a | G3 | 64 |
| T4_t1 | TNBC | Epi mono | ypT1b | ypNO (0/8) | G3 | 55 |
| T6_t1 | TNBC | 4x EC/ 12x P | ypT1b | ypNO (0/1) | G2 | 36 |

That is, in the context of this study a subject/patient was classified as a NR (or non-responder post-op (post-operation)), if the subject/patient had not responded or only partially responded to the neoadjuvant breast cancer therapy in particular NACT (all patients in this class were > ypTO), as assessed post-operatively, after completion of therapy.

In addition, as a comparative cohort, we analysed serum samples from 21 healthy female volunteers.

**Table 3: Control cohort**

| **Control cohort** | **age** |
|---|---|
| BL-10 | 83 |
| BL-16 | 59 |
| BL-15 | 65 |
| BL-31 | 46 |
| BL-14 | 79 |
| BL-20 | 65 |
| BL-38 | 53 |
| BL-33 | 35 |
| BL-09 | 69 |
| BL-26 | 63 |
| BL-40 | 38 |
| BL-04 | 24 |
| BL-36 | 32 |
| BL-12 | 46 |
| BL-18 | 62 |
| BL-22 | 73 |
| BL-27 | 50 |
| BL-05 | 41 |
| BL-13 | 52 |
| BL-34 | 76 |
| BL-11 | 54 |

The following timepoints were examined:

**Table 4: timepoints examined in the study**

| **Timepoints** | **Therapy cycle** |
|---|---|
| T0 | before start of the first cycle |
| T1 | Just before start of the third cycle (i.e. after 2x E+C and just before the 3^{rd} dose) |
| T3 | Just before start of the seventh cycle (i.e. after 4x EC + 6x paclitaxel (3x once weekly doses per cycle of paclitaxel) and just before the 7^{th} dose of paclitaxel |

The aim of the study was to detect a proteomic profile or individual proteins as markers for a therapy response.

The difficulty of proteome analysis from the medium "serum" is that approx. 22 proteins cover 99 % of the protein mass in the serum (6,7). Contrary to this are the more than 10,000 proteins that have been identified in serum so far. The probability of detection by mass spectrometry depends directly on the frequency of the peptide in the sample. Therefore, the insight into the diversity of the serum proteome is masked due to the highly abundant proteins and their peptides. To increase the probability of identifying low-abundant peptides, 14 high-abundant proteins were depleted within this study using an immunoaffinity kit. The proteins were then digested by the proteases Lys-C and trypsin and desalted on PreOmics columns.

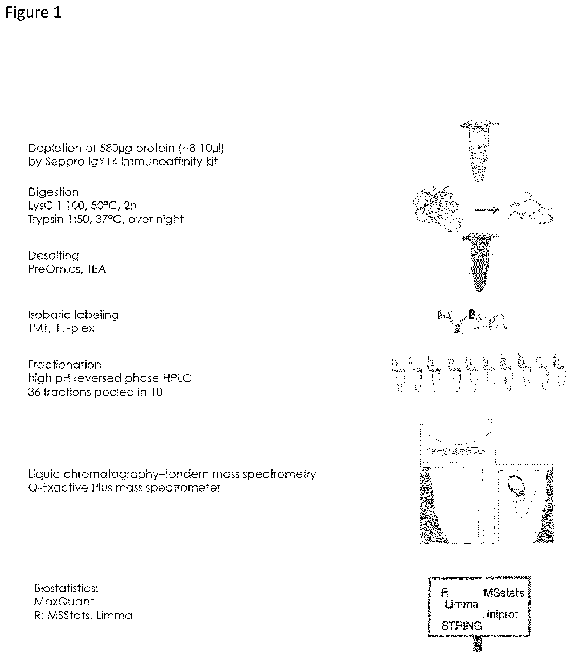

This was followed by isobaric labelling and fractionation via high pH reversed phase liquid chromatography HPLC. The resulting 36 fractions per run were combined into 10 fractions and then measured with liquid chromatography - tandem mass spectrometry LC-MS / MS (Figure 1).

The measured spectra were analysed with the MaxQuant program and assigned to the respective sample by identifying the label. Further biostatistical evaluation was carried out with the programming language R.

### Results

Within the present study, the present inventors were able to demonstrate a significant reaction to the NACT in the proteomic profile at timepoint T1 and T3 compared to timepoint T0.

### Comparison of T1 to T0 in the group of complete responders

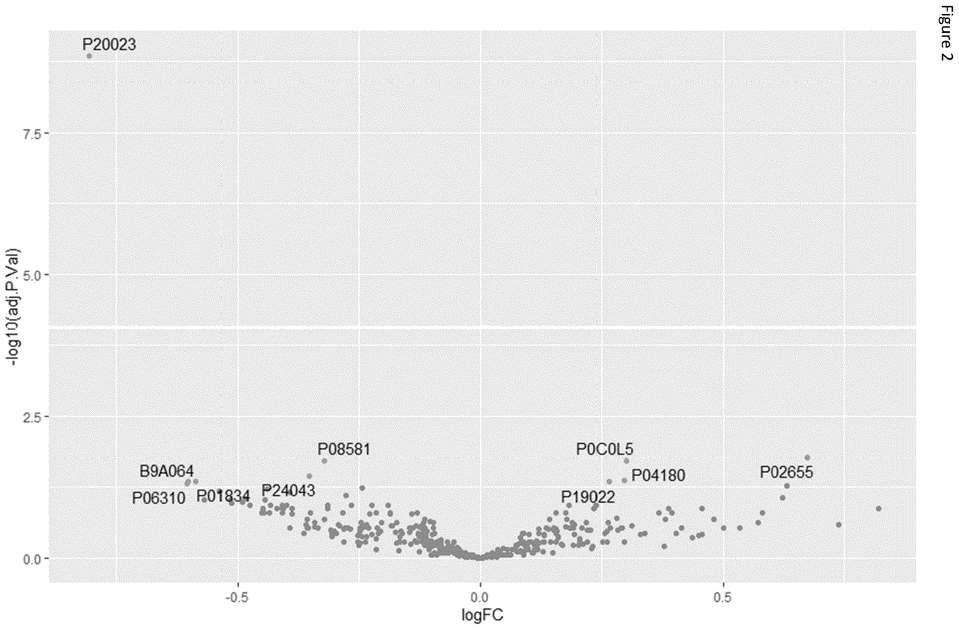

Figure 2 shows a comparison of T1 to T0 in complete responder (CR). The proteins show significant changes in their relative intensity (red dots). Proteins showing a significant downregulation in T1 compared to T0 are listed in Table 4 below. Proteins showing a significant upregulation in T1 compared to T0 are listed in Table 5 below.

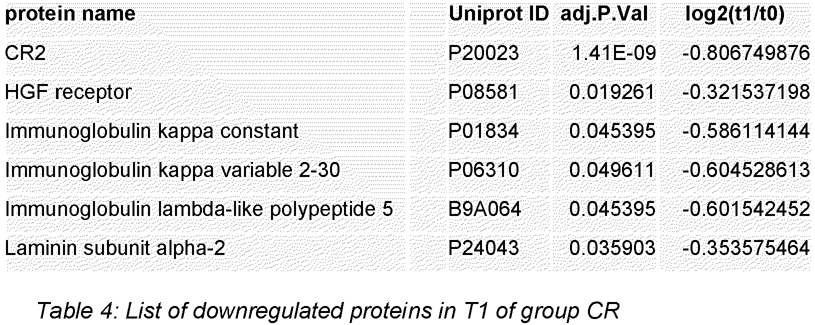

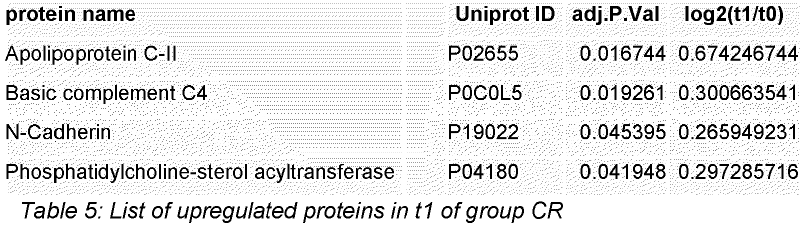

### Comparison of T1 to T0 in the group of non-responders post-op (NR)

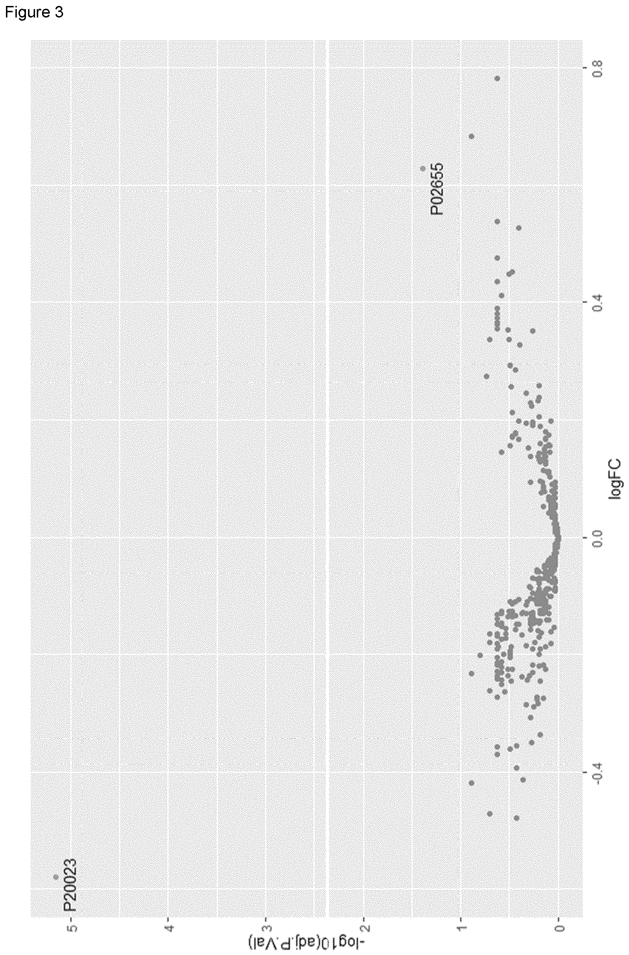

Figure 3 shows a comparison of T1 to T0 in non-responder post-op (NR). The proteins show significant changes in their relative intensity (red dots). Proteins showing a significant downregulation in T1 compared to T0 are listed in Table 6 below. Proteins showing a significant upregulation in T1 compared to T0 are listed in Table 7 below.

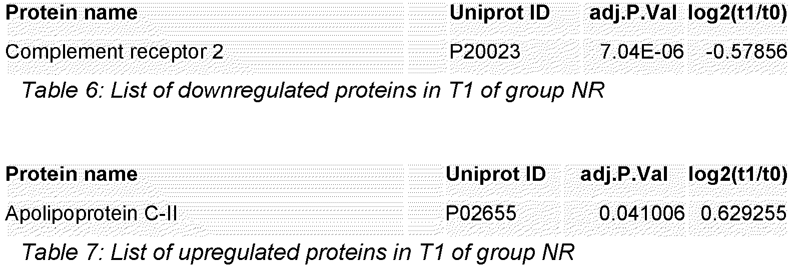

### Comparison of T3 to T0 in the group of complete responders

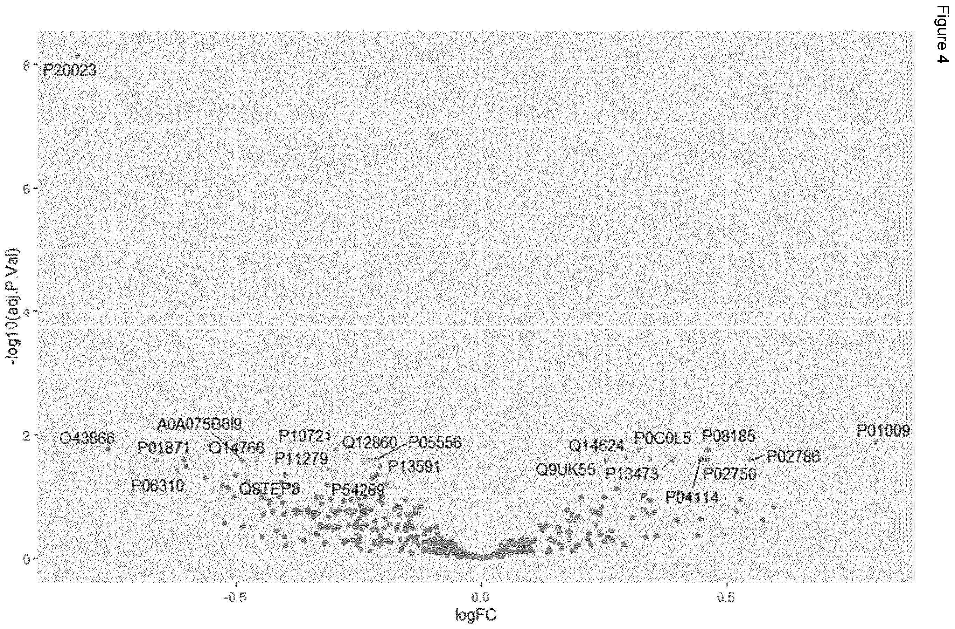

Figure 4 shows a comparison of T3 to T0 in complete responder (CR). The proteins show significant changes in their relative intensity (red dots). Proteins showing a significant downregulation in T3 compared to T0 are listed in Table 8 below. Proteins showing a significant upregulation in T3 compared to T0 are listed in Table 9 below.

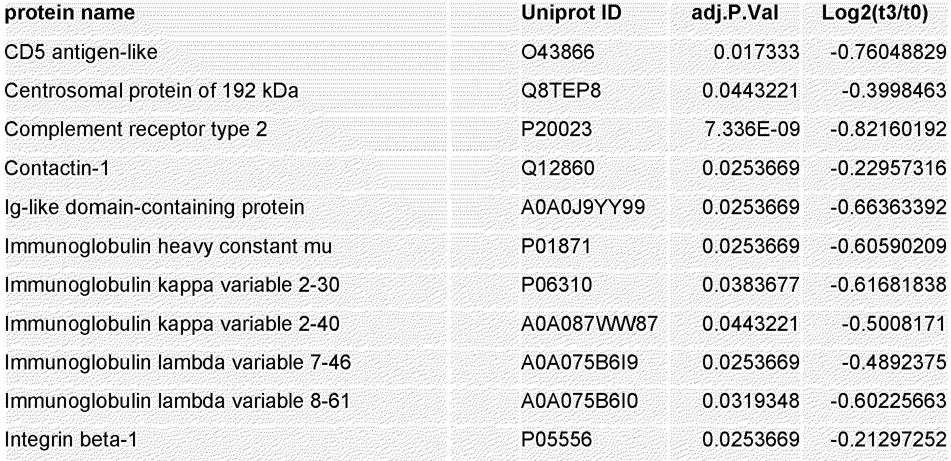

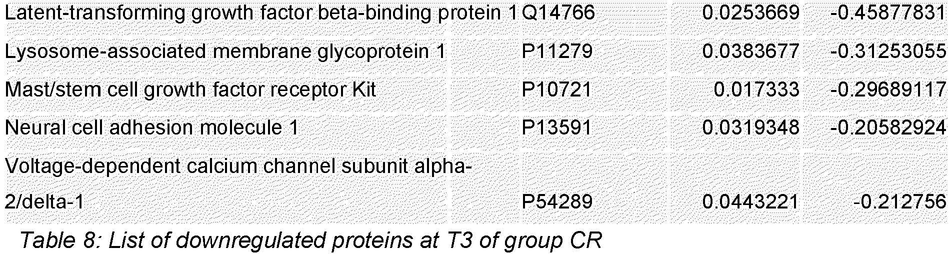

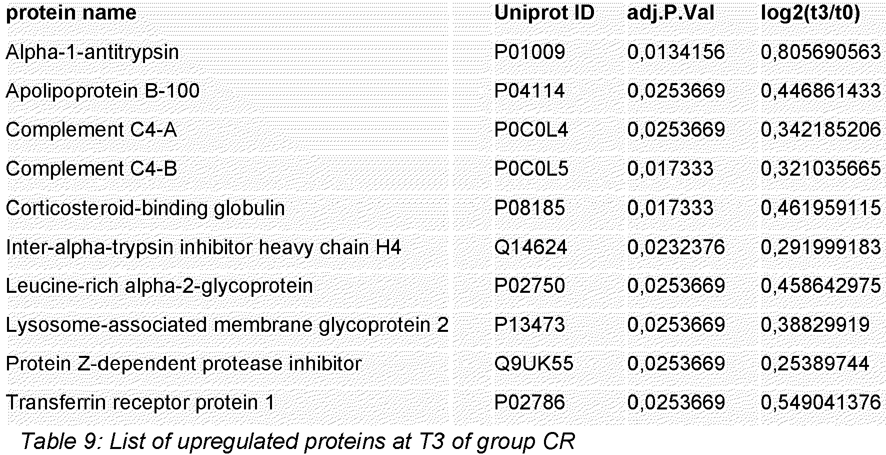

### Comparison of T3 to T0 in the group of non-responders post-op (NR)

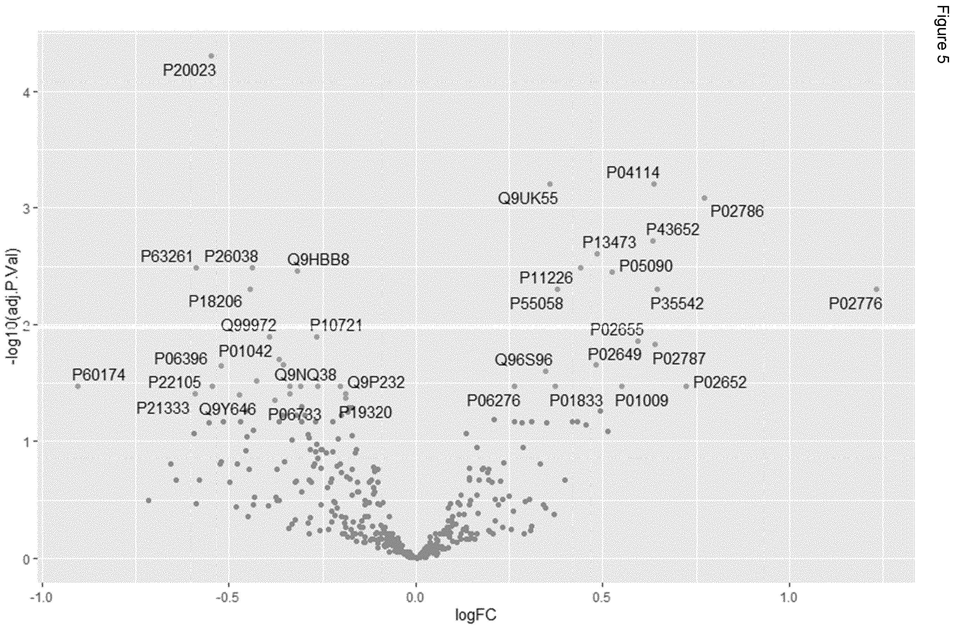

Figure 5 shows a comparison of T3 to T0 in non-responder post-op (NR). The proteins show significant changes in their relative intensity (red dots). Proteins showing a significant downregulation in T1 compared to T0 are listed in Table 10 below. Proteins showing a significant upregulation in T1 compared to T0 are listed in Table 11 below.

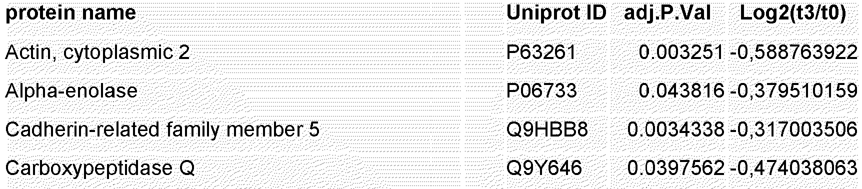

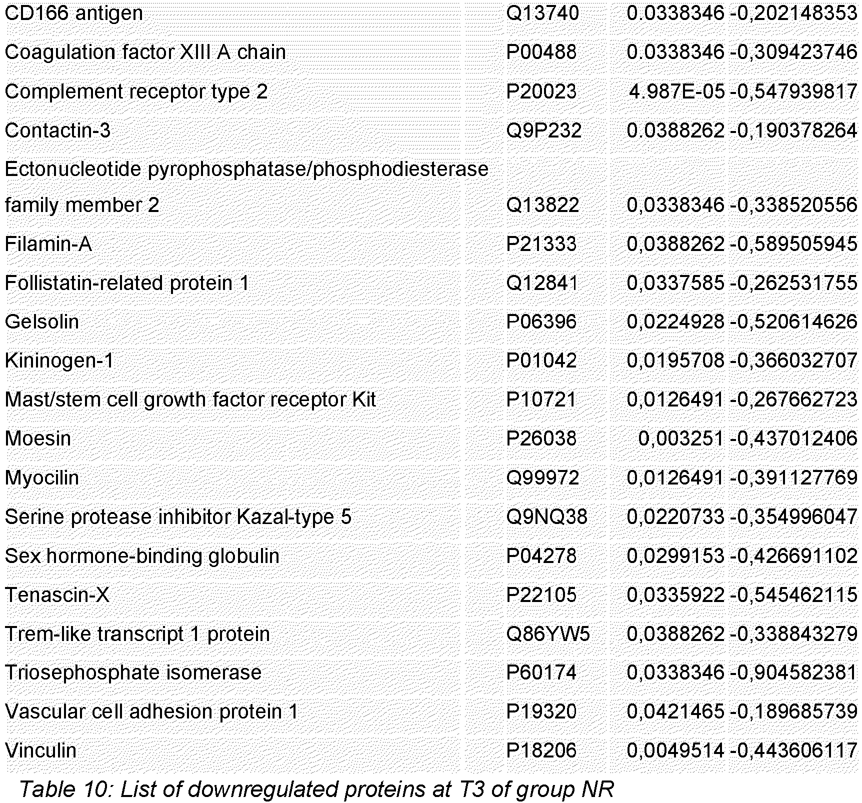

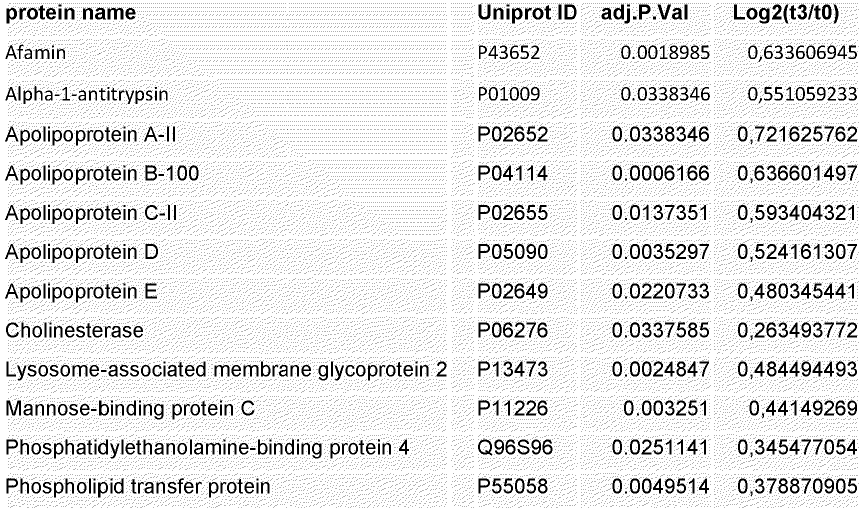

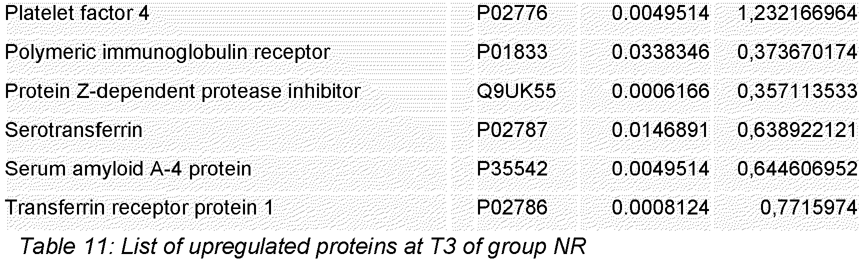

### Marker candidates

### Proteins with different behaviour in CR and NR

### HGF-Rezeptor

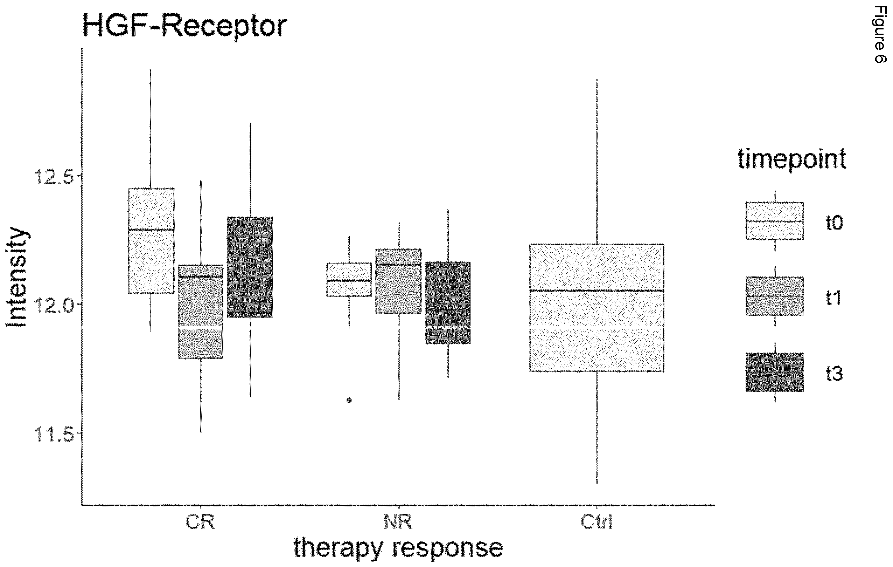

Figure 6 shows the relative Intensity of the HGF receptor over time, in comparison to the control (Ctrl) cohort. Over time, there is a significant decrease in the relative intensity of the HGF receptor at timepoint T1 in the CR group compared to stable behaviour in the NR group.

### N-Cadherin

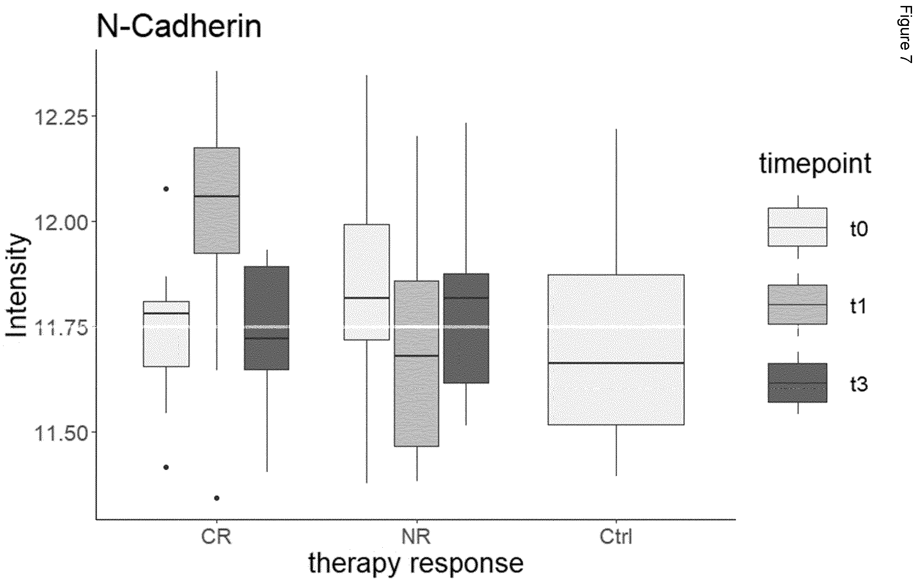

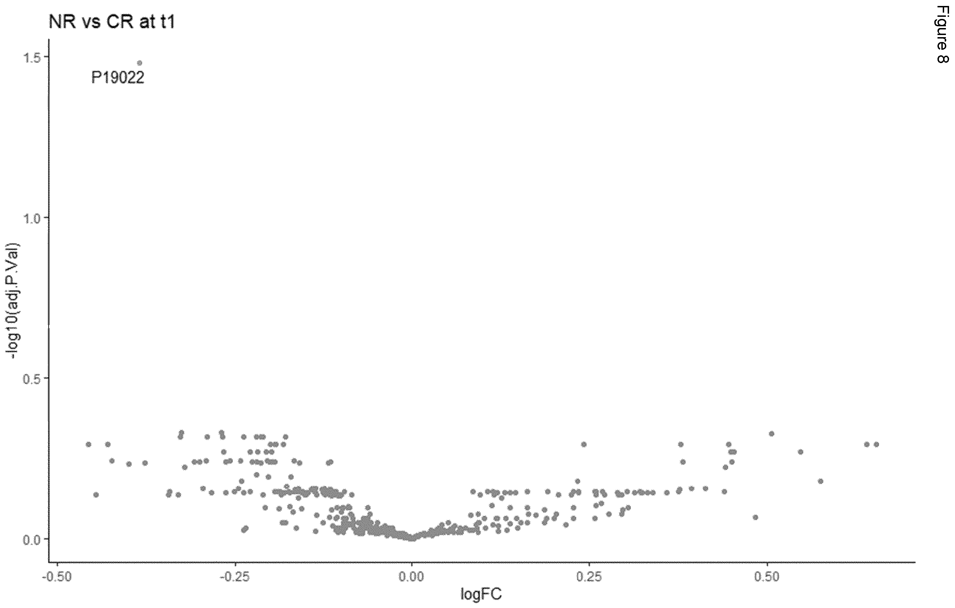

Figure 7 shows the relative Intensity of N-cadherin over time, in comparison to the control (Ctrl) cohort. An inverse behaviour between the groups CR and NR over time can be seen. In the CR group, there was a significant increase in the relative intensity of N-cadherin at timepoint t1, whereas the relative intensity at timepoint t1 decreased in the NR group. This is further shown in Figure 8, which shows a comparison of NR at T1 vs CR at T1; with the significant change in relative intensity shown as red dot (see also Table 12 below).

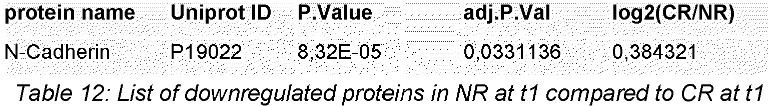

### Centrosomal protein 192 kDa

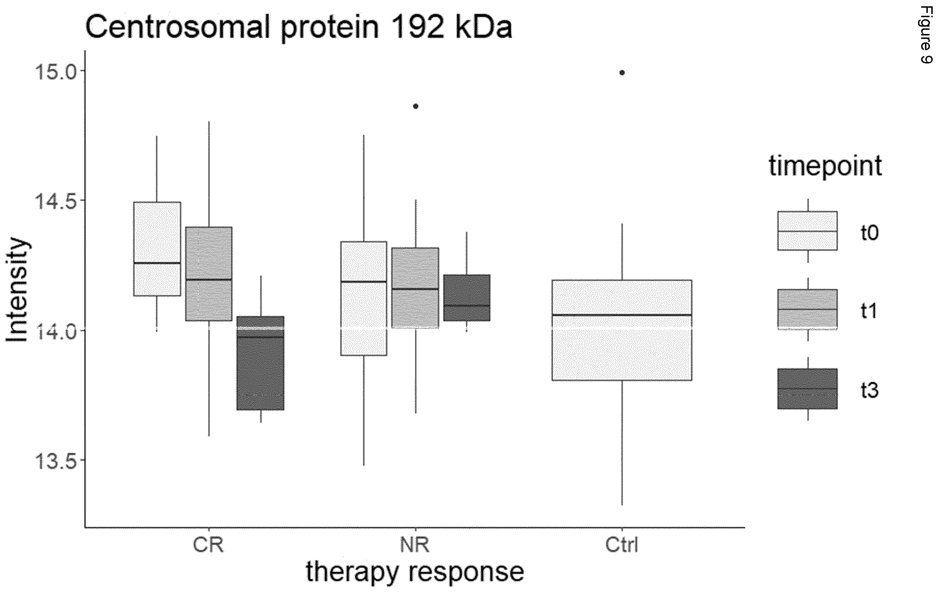

Figure 9 shows the relative intensity of centrosomal protein 192 kDa over time, in comparison to the control (Ctrl) group. There is a decrease in the relative intensity at timepoint T3 in the CR group. The level of the relative intensity is constant over timepoint in the group of NR.

### Contactin-1

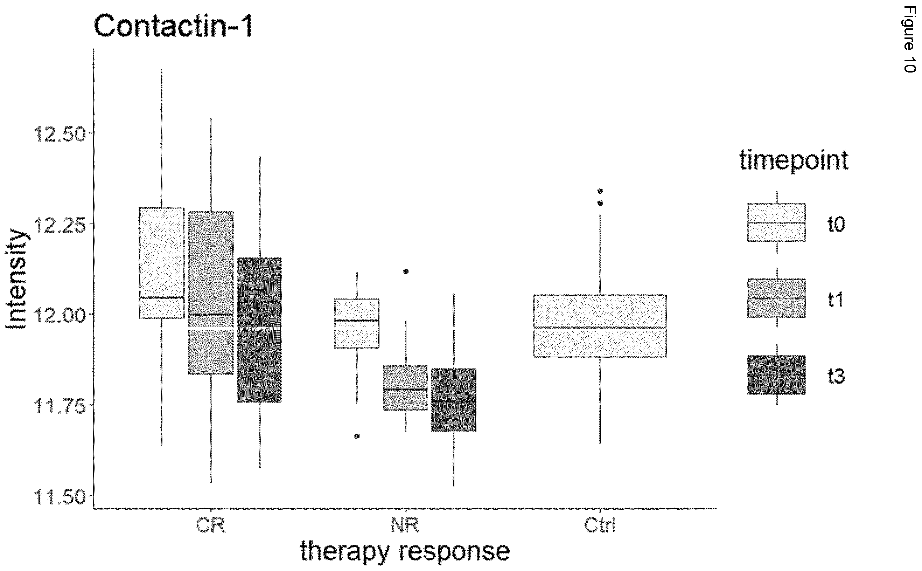

Figure 10 shows the relative intensity of contactin-1 over time, in comparison to the control (Ctrl) group. There is a decrease at timepoint T3 in the group of NR. The level of the relative intensity is constant over time in the CR group.

### Cholinesterase

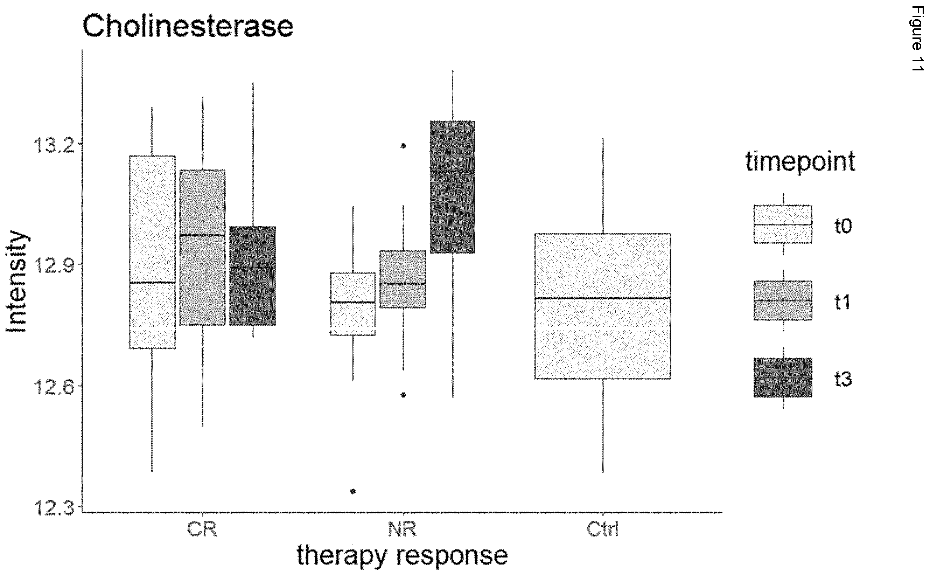

Figure 11 shows the relative intensity of cholinesterase over time, in comparison to to the control group. Over time there is an upregulation of the relative intensity at timepoint T3 in the group of NR. In the CR group, the relative intensity remains constant.

### Immunoglobulins

A range of proteins, and in particular several immunoglobulin-components, showed significant changes in their relative intensity. In addition, over time the present inventors were also able to determine a tendency towards inverse regulation between the groups CR and NR.

### Summary and possible applications

In this serum proteome study, it was possible to show an effect of neoadjuvant chemotherapy for breast cancer on various proteins. In addition, five potential marker candidates were identified, which show a different intensity behavior between the groups NR and CR. In this context, N-cadherin is emphasized, due to an inverse behavior comparing T0 to T1 and a significant difference in intensity between the two groups (NR and CR) at T1.

As possible markers for an (early) therapy response, the listed candidates can provide information about the pCR prognosis and thus lead to an individual therapy adjustment.

### Abbreviations

adj.P.Val = adjusted P value
BC = breast cancer
BSC = breast-conserving surgery
Cep192 = Centrosomal protein of 192 kDa
Ctrl = Control, in the present invention preferably from healthy subjects
ER = estrogen receptor
HGFR = hepatocyte growth factor receptor
HER2 = human epidermal growth factor receptor 2
Liquid chromatography-tandem mass spectrometry (LC-MS/MS)
LumB[HER2-] = Luminal B [human epidermal growth factor receptor 2-negative] breast cancer
Log2(Tx/Ty) = log2 of fold change in intensity (arb. unit) at timepoint x relative to timepoint y
log(FC) = Log Fold Change
NACT = neoadjuvant chemotherapy
non-responder = subject/patient not responding to the neoadjuvant breast cancer therapy, in particular NACT
PR = progesterone receptor
pCR = pathological complete remission
1 cycle of epirubicin + cyclophosphamide (E+C) = 2-3 weeks long, consisting of 1 dose of E+C followed by 2 or 3 weeks without treatment
1 cycle of paclitaxel = 3 weeks long consisting of 3 doses of paclitaxel once weekly (i.e. on days 1, 8 and 15)
T0 or t0 = timepoint just before start of the first cycle of neoadjuvant breast cancer therapy
T1 or t1 = timepoint just before start of the third cycle of E+C
T3 or t3 = timepoint just before start of the seventh cycle of the NACT of the study (i.e. after four cycles of E+C plus two cycles of paclitaxel)
responder = subject/patient responding to the neoadjuvant breast cancer therapy, in particular NACT
R0 resection = removal of cancer
TNBC = triple-negative breast cancer (TNBC)

### References

1. von Minckwitz, G., et al., Impact of treatment characteristics on response of different breast cancer phenotypes: pooled analysis of the German neo-adjuvant chemotherapy trials.Breast Cancer Res Treat, 2011. 125(1): p. 145-56.
2. Cortazar, P., et al., Pathological complete response and long-term clinical benefit in breast cancer: the CTNeoBC pooled analysis.Lancet, 2014. 384(9938): p. 164-72
3. von Minckwitz G, Blohmer JU, Costa SD, Denkert C, Eidtmann H, Eiermann W, Gerber B, Hanusch C, Hilfrich J, Huober J, et al. Response-guided neoadjuvant chemotherapy for breast cancer. J Clin Oncol. 2013;31:3623-3630. doi: 10.1200/JCO.2012.45.0940.
4. von Minckwitz, G., et al., In vivo chemosensitivity-adapted preoperative chemotherapy in patients with early-stage breast cancer: the GEPARTRIO pilot study.Ann Oncol, 2005. 16(1): p. 56-63
5. Statistisches Bundesamt (2013) Todesursachenstatistik, Fortschreibung des Bevölkerungsstandes: Sterbefälle, Sterbeziffern (je 100.000 Einwohner, altersstandardisiert) (ab 1998)
6. Wrotnowski, C., The future of plasma proteins. Gen Eng News, (1998) 18:14
7. Anderson NL, Anderson NG. The human plasma proteome: history, character, and diagnostic prospects. Mol Cell Proteomics. 2002 Nov;1(11):845-67. doi: 10.1074/mcp.r200007-mcp200. Erratum in: Mol Cell Proteomics. 2003 Jan;2(1):50. PMID: 12488461.
8. Leitlinienprogramm Onkologie (Deutsche Krebsgesellschaft, Deutsche Krebshilfe, AWMF): S3-Leitlinie Früherkennung, Diagnose, Therapie und Nachsorge des Mammakarzinoms, Version 4.4, 2021,AWMFRegisternummer:032-0450L,http://www.leitlinienprogramm-onkologie.de/leitlinien/mammakarzinom/(abgerufen am: 16.07.2021)

## Claims

1. A method for determining whether a subject is a responder or a non-responder to neoadjuvant breast cancer therapy, comprising determining the level of one or more marker proteins selected from the group consisting of N-cadherin; hepatocyte growth factor receptor (HGFR); centrosomal protein of 192 kDa (Cep192); contactin-1; cholinesterase; one or more immunoglobulin-components, selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61; and combinations thereof, and wherein the one or more marker proteins comprises HGFR,
a) in one or more serum samples of a subject undergoing neoadjuvant breast cancer therapy; and
b) in a comparative serum sample of that subject, or a comparative pooled serum sample from healthy subjects, or a comparative pooled serum sample from subjects suffering from breast cancer;
wherein a change in the level of the one or more marker proteins in one or more serum samples of a), relative to the level of the respective one or more marker proteins in the comparative serum sample of b), is indicative of the subject being a responder or a non-responder to neoadjuvant breast cancer therapy.

2. The method of claim 1, wherein the one or more marker proteins further comprises N-cadherin.

3. The method of claim 1 or 2, wherein the one or more marker proteins further comprises Cep192, contactin-1 and/or cholinesterase.

4. The method of any of claims 1 to 3, wherein the one or more marker proteins further comprises one or more immunoglobulin-components selected from the group consisting of Immunoglobulin kappa constant, Immunoglobulin kappa variable 2-30, Immunoglobulin lambda-like polypeptide 5, Ig-like domain-containing protein, Immunoglobulin heavy constant mu, Immunoglobulin kappa variable 2-40, Immunoglobulin lambda variable 7-46, and Immunoglobulin lambda variable 8-61.

5. The method according to any of the preceding claims, wherein the comparative serum sample of b) is a comparative serum sample of that subject, taken before or at the start of the neoadjuvant breast cancer therapy.

6. The method according to any of the preceding claims, wherein neoadjuvant breast cancer therapy comprises, or consists of, neoadjuvant chemotherapy (NACT).

7. The method according to claim 6, wherein the neoadjuvant chemotherapy (NACT) comprises, or consists of, administration of an anthracycline, in combination or sequentially administered with a taxane.

8. The method according to claim 6 or 7, wherein the NACT comprises, or consists of, administration of epirubicin and/or cyclophosphamide, and paclitaxel, administered in combination or sequentially.

9. The method according to any of the preceding claims,
- wherein the comparative serum sample of b) is a comparative serum sample of that subject taken before or at the start of the neoadjuvant breast cancer therapy; and/or
- wherein if the marker protein is N-Cadherin, HGFR, Immunoglobulin kappa constant, or Immunoglobulin lambda-like polypeptide 5, a serum sample of a) is a sample taken 2 to 12 weeks after start of the neoadjuvant breast cancer therapy; and/or
- wherein if the marker protein is Cep192, contactin-1, cholinesterase, Ig-like domain-containing protein, Immunoglobulin heavy constant mu, Immunoglobulin kappa variable 2-40, Immunoglobulin lambda variable 7-46, or Immunoglobulin lambda variable 8-61, the serum samples of a) is a sample taken 8 to 20 weeks after start of the neoadjuvant breast cancer therapy; and/or
- wherein if the marker protein is Immunoglobulin kappa variable 2-30, the serum samples of a) is a sample taken 2 to 20 weeks after start of the neoadjuvant breast cancer therapy.

10. The method according to any of the preceding claims,
- wherein if the marker protein is N-Cadherin, HGFR, Immunoglobulin kappa constant, or Immunoglobulin lambda-like polypeptide 5, a serum sample of a) is a sample taken after administration of a first dose but before administration of a fifth dose of neoadjuvant breast cancer therapy; and/or
- wherein if the marker protein is Cep192, contactin-1, cholinesterase, Ig-like domain-containing protein, Immunoglobulin heavy constant mu, Immunoglobulin kappa variable 2-40, Immunoglobulin lambda variable 7-46, or Immunoglobulin lambda variable 8-61, the serum samples of a) is a sample taken after administration of a sixth dose but before administration of a fourteenth dose of neoadjuvant breast cancer therapy; and/or
- wherein if the marker protein is Immunoglobulin kappa variable 2-30, the serum samples of a) is a sample taken after administration of a first dose but before administration of administration of a thirteenth dose of neoadjuvant breast cancer therapy.

11. The method according to any of the preceding claims, wherein, relative to the level of the respective protein marker in the comparative serum sample of b),
i. an increase in the protein level of N-cadherin in a serum samples of a) is indicative of the subject being a responder, and a decrease or a constant protein level of N-cadherin is indicative of the subject being a non-responder; and/or
ii. a decrease in the protein level of HGFR in a serum samples of a) is indicative of the subject being a responder; and a constant protein level or an increase is indicative of the subject being a non-responder; and/or
iii. a decrease in the protein level of Cep192 in a serum samples of a) is indicative of the subject being a responder, and a constant protein level or an increase is indicative of the subject being a non-responder; and/or
iv. a constant protein level of contactin-1 in a serum samples of a) or an increase is indicative of the subject being a responder, and a decrease is indicative of the subject being a non-responder; and/or
v. a constant protein level of cholinesterase in a serum samples of a) or a decrease is indicative of the subject being a responder, and an increase is indicative of the subject being a non-responder; and/or
vi. a decrease in the protein level of one or more immunoglobulin-components in one or more serum samples of a) is indicative of the subject being a responder, and a constant protein level or an increase is indicative of the subject being a non-responder.

12. The method of any of the preceding claims, wherein the protein level is determined by liquid chromatography-tandem mass spectrometry (LC-MS/MS).

13. The method of any one of the preceding claims, wherein the subject is a female human.

14. Method according to any of the preceding claims, wherein the subject suffers from triple negative breast cancer (TNBC) or Luminal B [human epidermal growth factor receptor 2-negative] (LumB[HER2-]) breast cancer.

15. The method of any one of the preceding claims, wherein the finding of the subject being a responder is predictive of the subject, after completion of neoadjuvant breast cancer therapy, achieving a pathological complete remission (pCR).

## Patentansprüche

1. Verfahren zum Bestimmen, ob ein Individuum ein Responder oder ein Nicht-Responder auf eine neoadjuvante Brustkrebstherapie ist, umfassend das Bestimmen des Spiegels von einem oder mehreren Markerprotein(en), ausgewählt aus der Gruppe, bestehend aus N-Cadherin; Hepatozytenwachstumfaktorrezeptor (HGFR); Zentrosomen-Protein 192 kDa (Cep192); Contactin-1; Cholinesterase; einer oder mehreren Immunglobulin-Komponente(n), ausgewählt aus der Gruppe, bestehend aus Immunglobulin-Kappa konstante Region, Immunglobulin-Kappa variable Region 2-30, Immunglobulin-Lambda-ähnlichem Polypeptid 5, Ig-ähnliche Domäne-enthaltendem Protein, Immunglobulin schwere konstante Region mu, Immunglobulin Kappa variable Region 2-40, Immunglobulin-Lambda variable Region 7-46 und Immunglobulin-Lambda variable Region 8-61 und Kombinationen davon, und wobei das eine oder die mehreren Markerprotein(e) HGFR,
a) in einer oder mehreren Serumprobe(n) eines Individuums, das sich einer neoadjuvanten Brustkrebstherapie unterzieht; und
b) in einer Vergleichsserumprobe von diesem Individuum oder einer Vergleichssammelserumprobe von gesunden Individuen oder einer Vergleichssammelserumprobe von Individuen, die an Brustkrebs leiden, umfasst;
wobei eine Veränderung des Spiegels des einen oder der mehreren Markerprotein(e) in einer oder mehreren Serumprobe(n) von a) in Bezug auf den Spiegel des jeweiligen einen Markerproteins oder der jeweiligen mehreren Markerproteine in der Vergleichsserumprobe von b) angibt, dass das Individuum ein Responder oder ein Nicht-Responder auf die neoadjuvante Brustkrebstherapie ist.

2. Verfahren nach Anspruch 1, wobei das eine oder die mehreren Markerprotein(e) ferner N-Cadherin umfasst/umfassen.

3. Verfahren nach Anspruch 1 oder 2, wobei das eine oder die mehreren Markerprotein(e) ferner Cep192, Contactin-1 und/oder Cholinesterase umfasst/umfassen.

4. Verfahren nach einem der Ansprüche 1 bis 3, wobei das eine oder die mehreren Markerprotein(e) ferner eine oder mehrere Immunglobulin-Komponente(n) umfasst/umfassen, die aus der Gruppe ausgewählt ist/sind, die aus Immunglobulin-Kappa konstante Region, Immunglobulin-Kappa variable Region 2-30, Immunglobulin-Lambda-ähnlichem Polypeptid 5, Ig-ähnliche Domäne-enthaltendem Protein, Immunglobulin schwere konstante Region mu, Immunglobulin Kappa variable Region 2-40, Immunglobulin-Lambda variable Region 7-46 und Immunglobulin-Lambda variable Region 8-61 besteht.

5. Verfahren nach einem der vorstehenden Ansprüche, wobei die Vergleichsserumprobe von b) eine Vergleichsserumprobe dieses Individuums ist, die vor oder zu Beginn der neoadjuvanten Brustkrebstherapie genommen wurde.

6. Verfahren nach einem der vorstehenden Ansprüche, wobei die neoadjuvante Brustkrebstherapie neoadjuvante Chemotherapie (NACT) umfasst oder daraus besteht.

7. Verfahren nach Anspruch 6, wobei die neoadjuvante Chemotherapie (NACT) die Verabreichung eines Anthracyclins in Kombination oder nacheinander mit einem Taxan umfasst oder daraus besteht.

8. Verfahren nach Anspruch 6 oder 7, wobei die NACT die Verabreichung von Epirubicin und/oder Cyclophosphamid und Paclitaxel in Kombination oder nacheinander umfasst oder daraus besteht.

9. Verfahren nach einem der vorstehenden Ansprüche,
- wobei die Vergleichsserumprobe von b) eine Vergleichsserumprobe des Individuums ist, die vor oder zu Beginn der neoadjuvanten Brustkrebstherapie genommen wurde; und/oder
- wobei, wenn das Markerprotein N-Cadherin, HGFR, Immunglobulin-Kappa konstante Region oder Immunglobulin-Lambda-ähnliches Polypeptid 5 ist, eine Serumprobe von a) eine Probe ist, die 2 bis 12 Wochen nach dem Beginn der neoadjuvanten Brustkrebstherapie genommen wurde; und/oder
- wobei, wenn das Markerprotein Cep192, Contactin-1, Cholinesterase, Ig-ähnliche Domäne-enthaltendes Protein, Immunglobulin schwere konstante Region mu, Immunglobulin-Kappa variable Region 2-40, Immunglobulin-Lambda variable Region 7-46 oder Immunglobulin-Lambda variable Region 8-61 ist, die Serumprobe von a) eine Probe ist, die 8 bis 20 Wochen nach dem Beginn der neoadjuvanten Brustkrebstherapie genommen wurde; und/oder
- wobei, wenn das Markerprotein Immunglobulin-Kappa variable Region 2-30 ist, die Serumprobe von a) eine Probe ist, die 2 bis 20 Wochen nach dem Beginn der neoadjuvanten Brustkrebstherapie genommen wurde.

10. Verfahren nach einem der vorstehenden Ansprüche,
- wobei, wenn das Markerprotein N-Cadherin, HGFR, Immunglobulin-Kappa konstante Region oder Immunglobulin-Lambda-ähnliches Polypeptid 5 ist, eine Serumprobe von a) eine Probe ist, die nach der Verabreichung einer ersten Dosis, aber vor der Verabreichung einer fünften Dosis der neoadjuvanten Brustkrebstherapie genommen wurde; und/oder
- wobei, wenn das Markerprotein Cep192, Contactin-1, Cholinesterase, Ig-ähnliche Domäne-enthaltendes Protein, Immunglobulin schwere konstante Region mu, Immunglobulin-Kappa variable Region 2-40, Immunglobulin-Lambda variable Region 7-46 oder Immunglobulin-Lambda variable Region 8-61 ist, die Serumprobe von a) eine Probe ist, die nach der Verabreichung einer sechsten Dosis, aber vor der Verabreichung einer vierzehnten Dosis der neoadjuvanten Brustkrebstherapie genommen wurde; und/oder
- wobei, wenn das Markerprotein Immunglobulin-Kappa variable Region 2-30 ist, die Serumprobe von a) eine Probe ist, die nach der Verabreichung einer ersten Dosis, aber vor der Verabreichung einer dreizehnten Dosis der neoadjuvanten Brustkrebstherapie genommen wurde.

11. Verfahren nach einem der vorstehenden Ansprüche, wobei, bezogen auf den Spiegel des jeweiligen Proteinmarkers in der Vergleichsserumprobe von b),
i. eine Erhöhung des Proteinspiegels von N-Cadherin in einer Serumprobe von a) angibt, dass das Individuum ein Responder ist, und ein gesenkter oder konstanter Proteinspiegel von N-Cadherin angibt, dass das Individuum ein Nicht-Responder ist; und/oder
ii. eine Senkung des Proteinspiegels von HGFR in einer Serumprobe von a) angibt, dass das Individuum ein Responder ist; und ein konstanter Proteinspiegel oder eine Erhöhung angibt, dass das Individuum ein Nicht-Responder ist; und/oder
iii. eine Senkung des Proteinspiegels von Cep192 in einer Serumprobe von a) angibt, dass das Individuum ein Responder ist; und ein konstanter Proteinspiegel oder eine Erhöhung angibt, dass das Individuum ein Nicht-Responder ist; und/oder
iv. ein konstanter Proteinspiegel von Contactin-1 in einer Serumprobe von a) oder eine Erhöhung angibt, dass das Individuum ein Responder ist, und eine Senkung angibt, dass das Individuum ein Nicht-Responder ist; und/oder
v. ein konstanter Proteinspiegel von Cholinesterase in einer Serumprobe von a) oder eine Senkung angibt, dass das Individuum ein Responder ist, und eine Erhöhung angibt, dass das Individuum ein Nicht-Responder ist; und/oder
vi. eine Senkung des Proteinspiegels von einer oder mehreren Immunglobulin-Komponente(n) in einer oder mehreren Serumprobe(n) von a) angibt, dass das Individuum ein Responder ist, und ein konstanter Proteinspiegel oder eine Erhöhung angibt, dass das Individuum ein Nicht-Responder ist.

12. Verfahren nach einem der vorstehenden Ansprüche, wobei der Proteinspiegel durch Flüssigchromatographie-Tandem-Massenspektrometrie (LC-MS/MS) bestimmt wird.

13. Verfahren nach einem der vorstehenden Ansprüche, wobei das Individuum ein weiblicher Mensch ist.

14. Verfahren nach einem der vorstehenden Ansprüche, wobei das Individuum an dreifach-negativem Brustkrebs (TNBC) oder Luminal-B [humaner epidermaler Wachstumsfaktorrezeptor 2-negativem] (LumB[HER2-])-Brustkrebs leidet.

15. Verfahren nach einem der vorstehenden Ansprüche, wobei die Erkenntnis, dass das Individuum ein Responder ist, vorhersagt, dass das Individuum nach Beendigung einer neoadjuvanten Brustkrebstherapie eine pathologische Komplettremission (pCR) erzielt.

## Revendications

1. Procédé permettant de déterminer si un sujet est un sujet répondant ou ne répondant pas à une thérapie néoadjuvante pour le cancer du sein, comprenant la détermination du niveau d'une ou plusieurs protéines marqueurs sélectionnées parmi le groupe constitué de la N-cadhérine, du récepteur de facteur de croissance hépatocytaire (HGFR) ; la protéine centrosomale de 192 kDa (Cep192) ; la contactine-1 ; la cholinestérase ; un ou plusieurs composants d'immunoglobuline, choisis dans le groupe constitué de la constante kappa d'immunoglobuline, la variable kappa 2-30 d'immunoglobuline, le polypeptide 5 de type lambda d'immunoglobuline, la protéine contenant un domaine de type Ig, la constante lourde mu d'immunoglobuline, la variable kappa 2-40 d'immunoglobuline, la variable lambda 7-46 d'immunoglobuline et la variable lambda 8-61 d'immunoglobuline ; et leurs combinaisons, et dans lequel la ou les protéines marqueurs comprennent le HGFR,
a) dans un ou plusieurs échantillons de sérum d'un sujet subissant une thérapie néoadjuvante pour le cancer du sein ; et
b) dans un échantillon de sérum comparatif de ce sujet, ou un échantillon de sérum comparatif regroupé provenant de sujets sains, ou un échantillon de sérum comparatif regroupé provenant de sujets atteints d'un cancer du sein ;
dans lequel un changement du niveau d'une ou plusieurs protéines marqueurs dans un ou plusieurs échantillons de sérum de a), par rapport au niveau de la ou les protéines marqueurs respectives dans l'échantillon de sérum comparatif de b), indique si le sujet est un sujet répondant ou ne répondant pas à une thérapie néoadjuvante pour le cancer du sein.

2. Procédé selon la revendication 1, dans lequel la ou les protéines marqueurs comprennent en outre la N-cadhérine.

3. Procédé selon la revendication 1 ou 2, dans lequel la ou les protéines marqueurs comprennent en outre la Cep192, la contactine-1 et/ou la cholinestérase.

4. Procédé selon l'une quelconque des revendications 1 à 3, dans lequel la ou les protéines marqueurs comprennent en outre un ou plusieurs composants d'immunoglobuline choisis dans le groupe constitué de la constante kappa d'immunoglobuline, la variable kappa 2-30 d'immunoglobuline, le polypeptide 5 de type lambda d'immunoglobuline, la protéine contenant un domaine de type Ig, la constante mu lourde d'immunoglobuline, la variable kappa 2-40 d'immunoglobuline, la variable lambda 7-46 d'immunoglobuline et la variable lambda 8-61 d'immunoglobuline.

5. Procédé selon l'une quelconque des revendications précédentes, dans lequel l'échantillon de sérum comparatif de b) est un échantillon de sérum comparatif de ce sujet, prélevé avant ou au début de la thérapie néoadjuvante pour le cancer du sein.

6. Procédé selon l'une quelconque des revendications précédentes, dans lequel la thérapie néoadjuvante pour le cancer du sein comprend ou consiste en une chimiothérapie néoadjuvante (NACT).

7. Procédé selon la revendication 6, dans lequel la chimiothérapie néoadjuvante (NACT) comprend ou consiste en l'administration d'une anthracycline, en association ou administrée séquentiellement avec un taxane.

8. Procédé selon la revendication 6 ou 7, dans lequel la NACT comprend ou consiste en l'administration d'épirubicine et/ou de cyclophosphamide et de paclitaxel, administrés en association ou séquentiellement.

9. Procédé selon l'une quelconque des revendications précédentes,
- dans lequel l'échantillon de sérum comparatif de b) est un échantillon de sérum comparatif prélevé sur ce sujet avant ou au début de la thérapie néoadjuvante pour le cancer du sein ; et/ou
- dans lequel, si la protéine marqueur est la N-cadhérine, le HGFR, la constante kappa de l'immunoglobuline ou le polypeptide 5 de type lambda d'immunoglobuline, un échantillon de sérum a) est un échantillon prélevé 2 à 12 semaines après le début de la thérapie néoadjuvante pour le cancer du sein ; et/ou
- dans lequel, si la protéine marqueur est Cep192, la contactine-1, la cholinestérase, la protéine contenant un domaine de type Ig, la constante lourde mu d'immunoglobuline, la variable kappa 2-40 d'immunoglobuline, la variable lambda 7-46 d'immunoglobuline ou la variable lambda 8-61 d'immunoglobuline, les échantillons de sérum de a) sont des échantillons prélevés 8 à 20 semaines après le début de la thérapie néoadjuvante pour le cancer du sein ; et/ou
- dans lequel, si la protéine marqueur est la variable kappa 2-30 d'immunoglobuline, les échantillons de sérum de a) sont des échantillons prélevés 2 à 20 semaines après le début de la thérapie néoadjuvante pour le cancer du sein.

10. Procédé selon l'une quelconque des revendications précédentes,
- dans lequel, si la protéine marqueur est la N-cadhérine, le HGFR, la constante kappa immunoglobuline ou le polypeptide 5 de type immunoglobuline lambda, un échantillon de sérum de a) est un échantillon d' e prélevé après l'administration d'une première dose mais avant l'administration d'une cinquième dose de thérapie néoadjuvante pour le cancer du sein ; et/ou
- dans lequel, si la protéine marqueur est Cep192, la contactine-1, la cholinestérase, la protéine contenant un domaine de type Ig, la constante lourde mu d'immunoglobuline, la variable kappa 2-40 d'immunoglobuline, la variable lambda 7-46 d'immunoglobuline ou la variable lambda 8-61 d'immunoglobuline, les échantillons de sérum de a) sont des échantillons prélevés après l'administration d'une sixième dose mais avant l'administration d'une quatorzième dose de thérapie néoadjuvante pour le cancer du sein ; et/ou
- dans lequel, si la protéine marqueur est le variable kappa 2-30 d'immunoglobuline, les échantillons de sérum de a) sont des échantillons prélevés après l'administration d'une première dose mais avant l'administration d'une treizième dose d'une thérapie néoadjuvante pour le cancer du sein.

11. Procédé selon l'une quelconque des revendications précédentes, dans lequel, par rapport au niveau du marqueur protéique respectif dans l'échantillon de sérum comparatif de b),
i. une augmentation du niveau de protéine N-cadhérine dans un échantillon de sérum de a) indique que le sujet est un sujet répondant, et une diminution ou un niveau de protéine constant N-cadhérine indique que le sujet est un sujet non-répondant ; et/ou
ii. une diminution du niveau de protéine HGFR dans un échantillon de sérum de a) indique que le sujet est un sujet répondant ; et un niveau de protéine constant ou une augmentation indique que le sujet est un sujet non-répondant ; et/ou
iii. une diminution du taux de protéine Cep192 dans les échantillons de sérum de a) indique que le sujet est un sujet répondant, et un taux de protéine constant ou une augmentation indique que le sujet est un sujet non-répondant ; et/ou
iv. un taux constant de protéine contactine-1 dans les échantillons de sérum de a) ou une augmentation indique que le sujet est un sujet répondant, et une diminution indique que le sujet est un sujet non-répondant ; et/ou
v. un taux constant de protéine cholinestérase dans les échantillons de sérum de a) ou une diminution indique que le sujet est un sujet répondant, et une augmentation indique que le sujet est un sujet non-répondant ; et/ou
vi. une diminution du taux de protéines d'un ou plusieurs composants d'immunoglobulines dans un ou plusieurs échantillons de sérum de a) indique que le sujet est un sujet répondant, et un taux de protéines constant ou une augmentation indique que le sujet est un sujet non-répondant.

12. Procédé selon l'une quelconque des revendications précédentes, dans lequel le niveau de protéine est déterminé par chromatographie liquide-spectrométrie de masse en tandem (LC-MS/MS).

13. Procédé selon l'une quelconque des revendications précédentes, dans lequel le sujet est une femme.

14. Procédé selon l'une quelconque des revendications précédentes, dans lequel le sujet souffre d'un cancer du sein triple négatif (TNBC) ou d'un cancer du sein luminal B [récepteur 2 du facteur de croissance épidermique humain négatif] (LumB[HER2-]).

15. Procédé selon l'une quelconque des revendications précédentes, dans lequel le fait que le sujet soit un sujet répondant permet de prédire que le sujet, après avoir suivi une thérapie néoadjuvante pour le cancer du sein, obtiendra une rémission pathologique complète (pCR).
